# EUROPEAN PATENT SPECIFICATION

(11) **EP 1 898 706 B1**
(45) Date of publication and mention of the grant of the patent: **04.04.2018**
(21) Application number: 06813198.6
(22) Date of filing: 24.05.2006
(51) Int. Cl.: A01N 55/00, A01P 1/00

(54) **SELENIUM-BASED BIOCIDAL FORMULATIONS AND METHODS OF USE THEREOF**
BIOZIDFORMULIERUNGEN AUF SELENBASIS UND VERFAHREN ZU IHRER VERWENDUNG
FORUMULATIONS BIOCIDES A BASE DE SELENIUM ET PROCEDES D'UTILISATION

(30) Priority: 24.05.2005 US 683847 P; 23.05.2006 US 802670 P
(43) Date of publication of application: 19.03.2008
(73) Proprietor: Selenium, Ltd., Austin, TX 78738 (US)
(72) Inventor: REID, Ted. W., Austin, TX 78738 (US); SPALLHOLZ, Julian, E., Austin, TX 78738 (US)
(74) Representative: Richards, John
(86) International application number: PCT/US2006/020310
(87) International publication number: WO 2007/008293

(56) References cited:
- WO-A-00/49868
- WO-A-95/31218
- WO-A2-00/67762
- DD-A3- 210 516
- JP-A- 4 108 705
- JP-A- 4 120 055
- JP-A- 58 057 306
- US-A- 4 496 559
- US-A- 4 729 986
- US-A- 5 721 241
- ROSA R M ET AL: "Genotoxicity of diphenyl diselenide in bacteria and yeast" MUTATION RESEARCH. GENETIC TOXICOLOGY AND ENVIRONMENTAL MUTAGENESIS, ELSEVIER, AMSTERDAM, NL, vol. 563, no. 2, 10 October 2004 (2004-10-10), pages 107-115, XP004568012 ISSN: 1383-5718

## Description

### BACKGROUND OF THE INVENTION

### 1. Field of the Invention

The present invention relates generally to biocidal formulations that utilize free radical generation as a mechanism of toxicity, and more specifically, to selenium-based formulations that utilize free radical generation as a mechanism of toxicity.

### 2. Description of the Background Art

Selenium (Se) is among the most toxic of all known minerals. Its toxicity symptoms in horses were most likely described by Marco Polo while traveling the silk road in China. In the 1920's, loss of livestock in parts of the western and central United States was severe. Those losses of livestock were investigated by the United States Department of Agriculture Experiment Station in South Dakota. In 1934, the cause of the loss of livestock was traced by the Experiment Station to the element selenium which was high in certain soils and high secondarily in plants from several species of *Astragalus* (vetch), *Xylorrhiza* (woody aster), *Conopsis* (goldenrod) and *Stanleya* (Prince's Plume). Ingestion of these and other Se containing plants by livestock often proved to be fatal.

Throughout the period of time between the discovery of selenium toxicity in livestock in 1934 and 1988, many hypotheses were put forth to explain the mechanism by which many but not all compounds of selenium were toxic. None of these theories of selenium toxicity proved satisfactory in fully explaining why selenium was toxic. In 1989, Seko et al. (In: Proceedings of the fourth international symposium on selenium and medicine (ed., Wendel, A.) pp.70-73, Springer-Verlag, Heidelberg, Germany, (1989)), reported that selenite, (SeO₃), an inorganic form of Se, reacted with a thiol, glutathione, (GSH), to produce superoxide (O₂⁻). Since superoxide is a known toxicant, this raised the possibility that all selenium compounds that are toxic might generate superoxide. Through the testing of many selenium compounds, it was found that the inorganic compounds, SeO₃ and selenium dioxide (SeO₂) were able to generate O₂⁻ and hydrogen peroxide (H₂O₂) when presented with a thiol, such as glutathione, cysteine (CysSH), or dithiothreitol D(SH)₂. Furthermore, it was found that all diselenides tested of the composition RSeSeR likewise would generate O₂⁻ and H₂O₂ when presented with any of the before mentioned thiols.

In 1947, Feigl et al. (Analytical Chemistry, 19:351-353 (1947)), reported that selenium could catalyze a redox reaction involving sulfide oxidation. This soon became a common test for selenium using methylene blue. This reaction was further studied by others using different selenium compounds and thiols, demonstrating catalysis for some but not all selenium compounds. See, West et al. (Analytic Chemistry, 40:966-968 (1968)); Levander et al. (Biochemistry, 12:4591-4595 (1973)), Rhead et al. (Biorganic Chemistry, 3:225-242 (1974)). The selenium catalytic activity of selenocystine (RSeSeR) in the presence of thiols was reported in 1958. It is now believed that all of the foregoing reactions of selenium compounds produce superoxide. See, Xu et al. (Advances in Free Radical Biology and Medicine, 1:35-48 (1991)); Xu et al. (Huzahong Longong Daxus Xuebao, 19:13-19 (1991)); Kitahara et al. (Archives of toxicology, 67:.497-501 (1993)); Chaudiere et al. (Archives of Biochemistry and Biophysics, 296:328-336 (1992)).

Selenium and a number of its compounds have been known since the early 1970's to possess anti-cancer properties. It has been generally recognized that selenite and selenium dioxide are good anti-cancer agents *in vitro* and in experimental animals and that the compounds are also cytotoxic to both cancer and normal cells *in vitro.* U.S. Pat. No. 5,104,852 issued to Kralick et al. describes the use of selenodiglutathione and other selenodithiols of the configuration (GSSeSG) to treat cancer. Selenodiglutathione is the product of the reaction between selenite or selenium dioxide with glutathione. The compound, selenodiglutathione, has been isolated. U.S. Pat. No. 5,104,852, however, does not describe the mechanism of action by which selenodiglutathione and like compounds are useful in treating cancer.

In 1982, the interaction of selenite and selenocystine with glutathione in the cytotoxicity and lysis of rat erythrocyte membranes was described by Hu et al. (Biochemical Pharmacology, 32:857-961 (1983)). This cytotoxicity, as revealed by scanning electron microscopy of rat erythrocytes, caused the erythrocyte membranes to become burred, the cells to quadruple in size and lyse similar to that described by Kellogg et al. (J. Biol. Chem., 252:6721-6728 (1977)). This toxicity, however, was not expressed by selenomethionine, a compound possessing the configuration RSeCH₃. In 1991, an article by Yan et al. (FASEB J., 5:A581 (1991)), showed a dose responsive toxicity of several selenium compounds to a human mammary tumor cell line. Additional investigations using lucigenin chemiluminescence and luminol chemiluminescence revealed a dose response in O₂⁻ and H₂O₂ generated chemiluminescence by selenite, selenium dioxide and all selenium compounds tested of the configuration RSeSeR. Furthermore, it was found that selenium compounds in the presence of either tumor cells or glutathione alone produced superoxide and H₂O₂. Chemiluminescence from the reactions of lucigenin with O₂⁻ or luminol with H₂O₂ could be quenched by the native enzymes superoxide dismutase (SOD), catalase (CT) or glutathione peroxidase (GSHPx). Denatured enzymes would not quench these reactions, confirming the generation of the free radical (O₂⁻) and H₂O₂ by selenium compounds and thiols. All of this selenium free radical chemistry has been reviewed by Spallholz (Free Radical Biology and Medicine, 17:45-64, (1994)).

A summation of this large body of experimental data on selenium toxicity, catalysis and carcinostatic activity is as follows:
1) The selenium compounds, SeO₂ and SeO₃, react with thiols to produce a selenodithiol of the configuration (RSSeSR). This compound is not toxic per se nor is it carcinostatic. The toxic carcinostatic form of RSeR is the reduced selenide anion, RSe⁻. This selenopersulfide form of Se is catalytic as shown by the inhibition of both catalysis and superoxide generation by iodoacetic acid and mercaptosuccinic acid.
2) Selenium compounds of the configuration (RSeSeR) or (RSeSeR') react with thiols to produce the reduced selenite anion RSe⁻ or R'Se⁻. This selenopersulfide form of Se is catalytic as shown by the inhibition of both catalysis and superoxide generation by iodoacetic acid and mercaptosuccinic acid.
3) Organic selenium catalysts of the configuration RSe⁻, the selenopersulfide anion, is catalytic in the presence of thiols, and RSe⁻ continues to generate superoxide (O₂⁻) ion as long as sufficient concentrations of O₂⁻ and thiol are in the medium. Selenium compounds derived from selenite or selenium dioxide reacting with glutathione (GSH) are converted to elemental selenium (Se·) as follows; SeO₃ (SeO₂)+2GSH→2GSSeSG→2GSSG+Se⁻. Elemental selenium (Se·) is non-catalytic and not toxic.
4) Compounds of selenium of the configuration RSe⁻ are toxic due to the catalytic acceleration of thiol oxidation which produces O₂⁻, H₂O₂ and the more toxic free radical, the hydroxyl radical (·OH). This chemistry had been discussed by Misra (J. Biol. Chem., 249:2151-2155 (1974)) for the spontaneous oxidation of thiols. The association of rapid thiol catalysis by selenium compounds of the configuration RSe⁻ and the toxicity from which it produced free radicals and reactive toxic oxygen products was recognized in 1992 by one of the inventors.

The use of selenium for the treatment of experimental cancer in animals and cancer in humans *in vivo* has been extensively described by many authors, such as Milner et al. ("Selenium and transplantable tumors," (Spallholz, J. E., Martin, J. L., Ganther, H. E., eds.) Selenium in Biology and Medicine, AVI Publishing Co. (1981)); Ip et al. ("Relationship between the chemical form of selenium and anticarcinogenic activity," CRC Press, Inc., pp.479-488 (1992)); Caffrey et al. (cancer Research, 52:4812-4816 (1992)); Schrauzer (Biol. Trace Elem. Res., 33:51-62 (1992)); and Yan et al. (Biochemical Pharmacology, 45:429-437 (1993)). The use of selenium as a cytotoxic agent to both normal cells and cancer cells *in vitro* for the injection of selenodiglutathione into a tumor mass to kill tumor cells has been described in U.S. Patent No. 5,104,852, issued to Kralick et al. In U.S. Pat. No. 4,671,958, Rodwell et al. described many antibacterial drugs, 3 antiviral drugs, 1 antifungal drug, 7 antineoplastic drugs, 3 radiopharmaceuticals, 3 heavy metals and 2 antimycoplasmals as drugs for antibody mediated delivery. The pharmacology for all of these drugs which are listed in Table 1 of U.S. Pat. No. 4,671,958 is generally understood. Table 1 of the Rodwell et al. patent does not contain selenium because its pharmacological action as a free radical generator of (O₂⁻) and other reactive oxygen molecules was not understood or known at that time.

Humans and other animals are in a constant immune-system battle with agents of infectious disease, such as bacteria, viruses, pathogenic fungi and protozoa. A particular problem for healthcare professionals dealing with these infectious agents has been the development of antibiotic resistant bacteria, which are refractory to many of the antibiotic agents that initially promised to provide a reliable cure.

A particularly critical problem for the healthcare industry has been the development and spread of infections within the hospital environment. While medical devices such as intravascular catheters provide a method for delivering fluids, medications, and nutrients to patients, their use is also frequently associated with hospital-spread infections; approximately 50% of hospital patients require intravenous access, and about 1-10% of catheters used eventually become contaminated. The consequences of such contamination range from phlebitis (venous inflammation) to sepsis (a systemic toxic condition resulting from the body-wide spread of bacteria and/or their products through the blood from the focus of infection).

Adhesive tapes used in conjunction with catheters and other medical devices are uniquely vulnerable to facilitating the spread of such infections in hospitals. This is because they are generally not washed or sterilized once they have been unpackaged, and, further, because a single roll of tape is generally used by several clinicians and on many different patients, and thereby becomes exposed to many different individuals. Furthermore, such adhesive tapes are frequently handled using ungloved hands and applied in close contact to the intravascular insertion site for extended periods of time. Indeed, one study found surprisingly high levels of infectious bacteria, including *Staphylococcus aureus,* on the outer layer of rolls of medical tape (3M TRANSPORE™) that were in use throughout a hospital in Toronto (see Redelmeier and Livesley (1999) J. Gen. Int. Med., 14:373-5).

As a result of widespread public concern with such infectious bacteria, antimicrobial materials such as fabrics, fibers, polymers and even children's toys have become increasingly popular. While the demand for such antimicrobial articles is high, relatively few types of such articles are available, and not all of those available are both effective against a broad spectrum of bacteria and capable of sustained antimicrobial activity without being released into the environment or being gradually chemically inactivated.

Research and development of durable functional fibers has advanced in recent years, including new methods of incorporating antibiotics as bactericidal agents directly into the fibers. The chemical and medical literature describes many compounds that have antimicrobial activity. Although the mechanism of action of these antimicrobials varies, they generally function by one or more of the following manners: inhibition of cell wall synthesis or repair; alteration of cell wall permeability; inhibition of protein synthesis; and inhibition of synthesis of nucleic acids (DNA or RNA).

At least since the 1870s, silver has been recognized as an antibacterial agent, and has been particularly noted for its ability to resist the development of drug-resistance in target bacteria. In general, silver cations (Ag+) are thought to possess antimicrobial activity because they are highly reactive chemical structures that bind strongly to electron donor groups containing sulfur, oxygen, or nitrogen that are present in microbial targets. The biological target molecules generally contain all these components in the form of thio, amino, imidazole, carboxylate, and phosphate groups. Silver ions act by displacing other essential metal ions such as calcium or zinc. The direct binding of silver ions to bacterial DNA may also serve to inhibit a number of important transport processes, such as phosphate and succinate uptake, and can interact with cellular oxidation processes as well as the respiratory chain. The silver ion-induced antibacterial killing rate is directly proportional to silver ion concentrations, typically acting at multiple targets. Indeed, for silver ion-based antimicrobial articles and devices to be effective as antimicrobial vectors, the silver ions with which they are impregnated must be slowly released into the environment so that they are free to contact and inhibit the growth of destructive microbes in the environment. Accordingly, the antimicrobial activity of silver-coated and silver-impregnated articles and devices is dependent upon the controlled release rate of the unbound, free silver ions they carry, and the continued antimicrobial efficacy of such silver-based antimicrobials is necessarily limited by the supply of free silver ions they retain.

The inventor's previous work, as disclosed and claimed in US Patent Nos. 5,783,454; 5,994,151; 6,033,917; 6,040,197; 6,043,098; 6,043,099; and 6,077,714; all issued to Spallholz et al., discloses methods for making selenium-carrier conjugates by covalently attaching (i) an organic selenium compound selected from the group consisting of RSeH, RSeR, RSeR', RSeSeR and RSeSeR', wherein R and R' are each an aliphatic residue containing at least one reactive group selected from the group consisting of aldehyde, amino, alcoholic, phosphate, sulfate, halogen or phenolic reactive groups and combinations thereof, to (ii) a carrier having a constituent capable of forming a covalent bond with said reactive groups of said selenium compound to produce a selenium-carrier conjugate which is capable of specific attachment to a target site. The carrier may be a protein, such as an antibody specific to a bacteria, virus, protozoa, or cell antigen, including without limitation, cell surface antigens, a peptide, carbohydrate, lipid, vitamin, drug, lectin, plasmid, liposome, nucleic acid or a non-metallic implantable device, such as an intraocular implant or a vascular shunt.

The '454 patent demonstrates the cytotoxicity of selenofolate of the configuration Folate-SeSeR, which produces superoxide in the presence of glutathione, as measured by lucigenin chemiluminescence; this modified vitamin compound is cytotoxic to cells upon uptake in a dose dependent manner. The '454 patent also demonstrates the ability of selenocystamine attached to plastic or a cellulose matrix to inhibit cellular growth.

However, the selenium-carrier conjugates of the prior art (as taught in the various patents listed above) require covalent attachment of the selenium compound to the carrier molecule in order to be effective, and the R and R' groups attached to the selenium must be aliphatic groups. In addition, the leaving groups generated when RSe⁻ is produced, as taught by the prior art, are toxic. Therefore there is a need for sustainable and effective biocidal agents that both avoid the formation of resistant microbes and can be adapted for use in solutions, suspensions and encapsulated particles, which overcome the disadvantages and defects of the prior art. It is to such improved biocidal compositions, and methods of production and use thereof, that the present invention is directed.

### SUMMARY OF THE INVENTION

The invention is based, in part, upon the finding that inorganic and organic selenium compounds, which catalyze the formation of free radical superoxide ions in the presence of oxygen and a reducing agent such a reduced thiol group or other electron donor, have biocidal activity when brought into contact with a species of interest, such as but not limited to, bacteria, viruses, mold, fungi, protozoa parasites, plant cells, animal cells, biological materials and combinations thereof. While not wishing to be bound by a single theory of their mechanism of biocial action, such selenium-containing compounds appear to provide for catalytic superoxide-mediated damage to a target species of interest by generating short-lived but highly reactive superoxide (O2-) ions in the presence of oxygen (O2) and reduced thiol groups (SH-groups) or other electron donating groups (i.e., Cofactors such as but not limited to, NADPH in NADPH dependent reductase) present on the target species of interest itself (e.g., from membrane proteins or other reducing sources present on or near the target species). Accordingly, the invention provides a method for preventing growth of a living cell and/or a living organism on a solid substrate comprising the steps of:
Providing solid substratem wherein the solid substrate is not a human or animal body;
Applying polymer matrix comprising an effective amount of a selenium compound to the solid matrix wherein the selenium compound is selected from the group consisting of-RSeH, RSeR', RSeSeR, RSeSeR', and RseX wherein. R and R' each are an aliphatic or phenolic residue, wherein R-comprises a reactive group that binds the selenium compound to the solid substrate via the polymer matrix and wherein X is a protecting group selected from the group consisting of a halogen, an imide, a cyanide, an azide, a phosphate, a sulfate, a nitrate, a carbonate, and selenium dioxide, wherein the selenium compound id non-covalently associated with the solid substrate via the polymer matrix and
Wherein the selenium compound comes into contact with and binds to a surface of the living cell and/or living organism, the selenium anion Se⁻ and the free radical species are formed, the free radical species inhibits and/or inactivates said living cell and/or living organism, thereby preventing growth of the living cell and/or living organism on the solid substrate, and wherein the selenium anion Se- remains attached to the surface via the polymer matrix after generation of the selenium anion Se⁻ and the free radicalspecies, such that selenium anion can continually generate additional free radiacl species when brought into contact with additional living cells and/or organisms.

The method of the present invention may be utilized in a method for preventing growth of a species of interest on an object or in a composition. The method includes applying an effective amount of any of the selenium compositions described above to an object or composition. In such method, the selenium compound is available to a surface of the species of interest to allow formation of the selenium anion Se- and free radical species. The selenium compound may be attached to the other components of the selenium composition by any non- covalent means known to a person having ordinary skill in the art, or the selenium compound may simply be present in a mixture or solution composition.

In one embodiment of the method of the present invention, the effective amount of the selenium compound is in a range of from about 0.01 [mu]g to about 100 [mu]g of elemental selenium per square centimeter of surface area.

It is an object of the invention to provide a new bacterialcidal and viralcidal agent. It is a further object of this invention to provide a methodology to use of the aforementioned free radical technology as bacterialcidal or viralcidal agents. The method of the present invention provides a means for directing the localized production of superoxide and descendant species thereof for selective destruction or modification of cells, tissue, membranes or extracellular fluids to combat a variety of localized problems, from infections, to cancer, to post surgical clotting and fibrosis.

Other objects, features and advantages of the present invention will become apparent from the following detailed description when read in conjunction with the accompanying figures and appended claims.

### BRIEF DESCRIPTION OF THE DRAWINGS

FIG. 1 illustrates targeted bacterial killing by selenium at 4°C.
FIG. 2 illustrates survival of bacteria expressing the F1 antigen at 4°C using 10 µM seleno-peptide.
FIG. 3 illustrates selenium peptide killing of bacteria at 25°C and 10 µM seleno- peptide concentration.
FIGS. 4A-4C illustrate both selenofolate concentration and time effects on breast cancer arrest in MDA-231 cells at 24 hours, 72 hours and 120 hours [0035] FIG. 5 demonstrates a dose response of MDA-231 breast cancer cells to selenite. FIG. 6 illustrates fluorescein staining data at 4 and 8 weeks. Data presented as mean ± standard error (SE).
FIG. 7A illustrates corneal epithelial thickness as recorded with a Corneo Gauge Plus pachymeter in LK mode. Data presented as mean ± SE. FIG. 7B illustrates total corneal thickness as recorded with a Corneo Gauge Plus pachymeter in RK mode. Data presented as mean ±SE.
FIG. 8 illustrates electron micrographs of corneas at low (2000x), middle (7000x), and high (20,000x) magnification. Magnification bars in the 4 left micrographs show 5 µm in 2 right micrographs. The corneas that wore treated lenses (Se) are in the top row and the corneas that wore control lenses (No Se) are in the bottom row.
FIG. 9 contains corneal histology slides (H&E stain - 230x). The cornea that wore the control lens (No Se) is on the left and the cornea that wore the Se-treated lens is on the right side (Se).
FIG. 10 contains low and high magnification electron micrographs of contact lenses placed in a broth inoculated with *P. aeruginosa.* The treated lens (S) is in the top row and the control lens (No Se) is in the bottom row.
FIG. 11 illustrates total protein deposition on control and treated lenses. Data presented as mean ± SE.
FIG. 12 illustrates relative lipid deposition on control and treated lenses. Data presented as mean ± SE.
FIG. 13 illustrates contact lenses after four days in broth of dividing *Staphylococcus aureus.* A: Selenium coated lens; B: control lens.
FIG. 14 illustrates human fibroblast growth in the presence of selenium-treated and untreated sponges.
FIG. 15 illustrates the ability of the selenium modified bonding agent, AAEMA, to generate superoxide radicals.
FIG. 16 illustrates inhibition of HIV with a peptide with and without selenium.

### DETAILED DESCRIPTION OF THE INVENTION

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The foregoing techniques and procedures are generally performed according to conventional methods well known in the art and as described in various general and more specific references that are cited and discussed throughout the present specification. See e.g., Sambrook et al. Molecular Cloning: A Laboratory Manual (2nd ed., Cold Spring Harbor Laboratory Press, Cold Spring Harbor, N.Y. (1989) and Coligan et al. Current Protocols in Immunology (Current Protocols, Wiley Interscience (1994)), which are incorporated herein by reference. The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

As utilized in accordance with the present disclosure, the following terms, unless otherwise indicated, shall be understood to have the following meanings:

The terms "covalently attached", "covalent bonding" and "covalent attachment" as used herein will be understood to refer to a stable chemical link between two atoms produced by sharing of one or more pairs of electrons. Covalent bonding is an intramolecular form of chemical bonding characterized by the sharing of one or more pairs of electrons between two components, producing a mutual attraction that holds the resultant molecule together. Atoms tend to share electrons in such a way that their outer electron shells are filled. Such bonds are always stronger than the intermolecular hydrogen bond and similar in strength to or stronger than the ionic bond. In contrast to the ionic and metallic bond, the covalent bond is directional, i.e. the bond angles have a great impact on the strength of the bond. Because of the directional character of the bond, covalently bound materials are more difficult to deform than metals.

The terms "noncovalently attached", "noncovalent bonding", "noncovalent interactions" and "noncovalent attachment" as used in accordance with the present invention will be understood to refer to any methods of attachment that do not involve a covalent attachment. A noncovalent bond is a chemical bond in which, in contrast to a covalent bond, no electrons are shared. Noncovalent bonds are relatively weak, but they can sum together to produce strong, highly specific interactions between molecules.

Noncovalent bonding refers to a variety of interactions that are not covalent in nature between molecules or parts of molecules that provide force to hold the molecules or parts of molecules together, usually in a specific orientation or conformation. Specific examples of non-covalent interactions include, but are not limited to, ionic bonds, hydrophobic interactions, hydrogen bonds, Van der Waals forces (aka London dispersion forces), Dipole-dipole bonds, and the like. "Noncovalent bonding", "Noncovalent interactions" and "Noncovalent forces" all refer to these forces as a whole without specifying or distinguishing which specific forces are involved because noncovalent interactions often involve several of these forces working in concert. Noncovalent bonds are weak by nature and must therefore work together to have a significant effect. In addition, the combined bond strength is greater than the sum of the individual bonds. This is because the free energy of multiple bonds between two molecules is greater than the sum of the enthalpies of each bond due to entropic effects.

The term "biocide" as utilized herein refers to a chemical substance capable of killing different forms of living organisms. A biocide can be a pesticide, such as but not limited to, fungicides, herbicides, insecticides, algicides, moluscicides, miticides, and rodenticides; or the biocide can be an antimicrobial, such as but not limited to, germicides, antibiotics, antibacterials, antivirals, antifungals, antiprotoas, and antiparasites.

The term "species of interest" as utilized in accordance with the present invention refers to any living cell(s) or organism that is killed or suppressed when exposed to free radicals. The term "species of interest" includes, but is not limited to, prokaryotes such as bacteria and archebacteria; viruses; eukaryotes such as mold, fungi, protozoa parasites, plant cells and animal cells; and biological materials such as proteins and nucleotides. Examples of prokaryotes include, but are not limited to, bacteria such as for example, *Staphylococcus aureus*, *Pseudomonas*, *Escherichia coli*, and *Bacillus subtilis.* Examples of viruses include, but are not limited to, Poxvirus, Papillomavirus, Filovirus, Bornavirus, Mimivirus, Picornavirus, Adenovirus, Retrovirus, Paramyxovirus, Flavivirus, Parvovirus, Hepadnavirus, Calcivirus, and Orthomyxovirus and Bacteriophage; specific viral examples include HIV, Rhinovirus, West Nile, Influenza, smallpox, and herpes simplex. Examples of parasites include, but are not limited to, arthropod parasites, helminth parasites, protozoal parasites, and hematoprotozoal parasites; specific examples include demodex mange, hookworm, and coccidia. Examples of eukaryotic cells include, but are not limited to, fibroblast cells, barnacles, epithelial cells, and cancer cells, including but not limited to, prostate cancer cells, breast cancer cells, leukemia, and lymphoma.

The terms "nucleotide" and "nucleic acid segment" as used herein shall mean a nucleotide of genomic, cDNA, or synthetic origin or some combination thereof, and thus includes naturally occurring nucleotides and modified nucleotides. The term "protein" referred to herein means a protein of cDNA, recombinant RNA, or synthetic origin or some combination thereof. The term "polypeptide" as used herein is a generic term to refer to native protein, fragments, or analogs of a polypeptide sequence. Hence, native protein, fragments, and analogs are species of the polypeptide genus.

The term "agent" is used herein to denote a chemical compound, a mixture of chemical compounds, a biological macromolecule, or an extract made from biological materials.

As used herein, the terms "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or enzymatic activity that can be detected by optical or calorimetric methods). In certain situations, the label or marker can also be therapeutic. Various methods of labeling polypeptides and glycoproteins are known in the art and may be used. Examples of labels for polypeptides include, but are not limited to, the following: radioisotopes or radionuclides (e.g., ³H, ¹⁴C, ¹⁵N, ³⁵S, ⁹⁰Y, ⁹⁹Tc, ¹¹¹In, ¹²⁵I, ¹³¹I), fluorescent labels (e.g., FITC, rhodamine, lanthanide phosphors), enzymatic labels (e.g., horseradish peroxidase, β-galactosidase, luciferase, alkaline phosphatase), chemiluminescent, biotinyl groups, predetermined polypeptide epitopes recognized by a secondary reporter (e.g., leucine zipper pair sequences, binding sites for secondary antibodies, metal binding domains, epitope tags). In some embodiments, labels are attached by spacer arms of various lengths to reduce potential steric hindrance.

The term "pharmaceutical agent or drug" as used herein refers to a chemical compound or composition capable of inducing a desired therapeutic effect when properly administered to a patient. Other chemistry terms herein are used according to conventional usage in the art, as exemplified by The McGraw-Hill Dictionary of Chemical Terms (Parker, S., Ed., McGraw-Hill, San Francisco (1985)), incorporated herein by reference).

The term "antineoplastic agent" is used herein to refer to agents that have the functional property of inhibiting a development or progression of a neoplasm in a human, particularly a malignant (cancerous) lesion, such as a carcinoma, sarcoma, lymphoma, or leukemia. Inhibition of metastasis is frequently a property of antineoplastic agents.

A "liposome" is a small vesicle composed of various types of lipids, phospholipids and/or surfactant. The components of the liposome are commonly arranged in a bilayer formation, similar to the lipid arrangement of biological membranes.

A "micelle" is an aggregate of surfactant molecules dispersed in a liquid colloid. Micelles are often globular in shape, but other shapes are possible, including ellipsoids, cylinders, bilayers, and vesicles. The shape of a micelle is controlled largely by the molecular geometry of its surfactant molecules, but micelle shape also depends on the conditions (such as temperature or pH, and the type and concentration of any added salt).

"Treatment" refers to both therapeutic treatment and prophylactic or preventative measures. Those in need of treatment include those already with the disorder as well as those in which the disorder is to be prevented.

A "disorder" is any condition that would benefit from treatment with the compositions of the present invention. This includes chronic and acute disorders or diseases including those pathological conditions which predispose the mammal to the disorder in question.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include squamous cell cancer, small-cell lung cancer, non-small cell lung cancer, gastrointestinal cancer, pancreatic cancer, glioblastoma, cervical cancer, ovarian cancer, liver cancer, bladder cancer, hopatoma, breast cancer, colon cancer, colorectal cancer, endometrial carcinoma, salivary gland carcinoma, kidney cancer, renal cancer, prostate cancer, vulval cancer, thyroid cancer, hepatic carcinoma and various types of head and neck cancer.

"Mammal" for purposes of treatment refers to any animal classified as a mammal, including human, domestic and farm animals, nonhuman primates, and zoo, sports, or pet animals, such as dogs, horses, cats, cows, etc.

The non-metal element selenium exists in several catalytic and non-catalytic oxidation states, *in vitro* and *in vivo.* If present in sufficient concentrations of thiol compounds, selenium compounds such as selenides, RSe-, oxidize thiols, producing superoxide (O₂⁻) and other biologically reactive oxygen species. Superoxide and the other produced reactive products, hydrogen peroxide, thiol radicals and other organic free radicals are toxic to biological membranes, molecules and cells. When present in sufficient concentration as the selenoselenide anion, RSe⁻, selenium can arrest and kill normal cells, cancer cells, bacterial cells, yeast cells and viruses. When organic selenium compounds are covalently attached to any targeting molecule such as a mono- or polyclonal antibody, peptide or polypeptide, hormone, vitamin, drug, or device, such conjugates comprise a new class of pharmaceuticals and devices that produce free radicals. Selenium is uniquely different from other elements that produce free radicals, i.e., iron, copper or cobalt, in that selenium can readily form small adducts replacing sulfur and it covalently combines with carbon and hydrogen compounds. Such selenium labeled adducts of the proper chemistry will remain non-toxic until activated by a thiol and the free radical pharmacology can be molecularly localized by the carrier molecule. This free radical chemistry is also useful for competitive protein binding assays. The free radical chemistry generated by selenium compounds can be detected by chemiluminescence or reduction of dyes by a spectrophotometer providing for quantitation of a compound which binds the antibody, hapten or drug to which selenium is attached and to which it subsequently reacts with thiols.

The present invention is related to selenium compositions of the configuration RSeH, RSeR, RSeR', RSeSeR, RseSeR', and more preferably, RSeX, for use as biocidal agents. Each of R and R' is an aliphatic residue containing at least one reactive group selected from the group consisting of aldehyde, amino, alcoholic, phosphate, sulfate, halogen, or phenolic reactive groups and combinations thereof. X is a protecting group that can be any electron withdrawing group known in the art; preferably, X is selected from the group consisting of a halogen, an imide, a cyanide, an azide, a phosphate, a sulfate, a nitrate, a carbonate, selenium dioxide, and combinations thereof. Specific examples include, but are not limited to, R-Se-Cl, R-Se-Br, R-Se-I, R-Se-CN, R-Se-N₃, R-Se-O-R", R-Se-S-R", R-Se-PO₃-R", R-Se-SO₃-R", and the like, wherein R" is H or an aliphatic residue. The protecting group can be removed once the selenium composition is produced and/or after the selenium composition is disposed in a solution, suspension or encapsulated molecule, or covalently attached. The protecting group can be removed *in vitro* or *in vivo,* if no toxic group is produced.

The Se-carrier conjugate thus produced is administered by injection, ingestion or topical cutaneous contact and carried by normal physiologic means to a target site or target molecule, or surgically implanted at a target site, whereupon superoxide (O₂⁻) is generated when the Se-carrier conjugate reacts with endogenous thiols on the surface of the targeted local tissue, bacteria, virus, protozoa or other targeted compounds. The selenium toxicity produced by the RSe- composition is very localized because it requires that a surface of a species of interest be available for interaction with the RSe- anion. R and R' are each selected from the group consisting of aliphatic residues containing one or more aldehyde, carboxylic, amino, alcoholic, phosphate, sulfate, halogen or phenolic reactive groups, and combinations thereof, such as --(CH₂)ₙHN₂, --(CH₂)ₙCOOH, --(CH₂)ₙ--Ø, wherein n is an integer greater than 1, and preferably between about 1 and 50, and more preferably between about 3 to 5. R and R' can be the same or different. The R groups themselves have no real role in the method of the invention, other than to provide reactive groups to bind to the carrier and to protect the selenium until it reaches the target sites. Accordingly, the length of the aliphatic chain is not important. The preferred molecular weight of the compound is about 1000 or less, but higher MWs will be suitable. Representative examples of selenium compounds include, but are not limited to NH₂CH₂CH₂SeCH₃ (RSeR'), NH₂CH₂CH₂SeCH₂CH₂NH₂ (RSeR), NH₂CH₂CH₂SeSeCH₂CH₂NH₂ (RSeSeR), NH₂CH₂CH₂SeSeCH₂CH₂NH-cellulose (RseSeR'), and NH₂CH₂CH₂SeCN (RSeX). The RSeX configuration is preferred. These selenium compounds, when brought into contact with thiol and oxygen, can generate superoxide (O₂⁻), H₂O₂ or hydroxyl radical (OH) or any other reactive oxygen species. The thiols can be exogenous thiols added for example, to a competitive immunoassay, endogenous thiols found in membranes, cellular cytoplasm or extracellular fluids. If native thiols are insufficient, exogenously supplied glutathione, glutathione derivatives, cysteine or other thiol or other electron donating molecules or atoms can be used expressly for the generation of superoxide. The selenium compositions of the present invention can be used to treat, in a pharmacological manner, cancer, both primary and metastatic; infections and diseases caused by (i) all viruses of all plant, animal or human origin; (ii) all bacteria of all plant, animal or human origin; (iii) all protozoans of all plant, animal or human origin; and (iv) other pathogens. The selenium compositions of the present invention, for example, when available to a surface of the virus, bacteria, protozoa or cancer cells, will catalyze the production of superoxide, H2O2 and other, reactive oxygen species. Viruses have surface proteins to which the selenium compositions of the present invention may come into close proximity. The selenium reacts with thiols in those surface proteins to generate the superoxide on the surface of the virus. The lack of an uptake mechanism in the virus is not important because the damage is done at the viral surface.

The selenium compounds used in the present invention may be non-covalently attached to the polymer matrix such as a cellulose pad, protein pad, other carbohydrate pad, plastic or other polymer matrix or a biocompatable matrix for the purpose of generating superoxide (O₂⁻) and its descendent reactive oxygen species when available to a surface of a species of interest. The device should not be metallic, but may be an organometallic compound or a metal coated with an organo compound to which the selenium compound can attach. The selenium non-covalently attached to the insoluble matrix inhibits cell growth in the localized area of the matrix due to the localized generation of superoxide.

In yet another alternative, the selenium compounds of the present invention may be suspended in a composition. Examples of compositions in which the selenium compounds of the present invention may be suspended include, but are not limited to, coatings, epoxies, glues, caulks, emulsions, and the like. Specific examples include matrix coatings, paints, adhesives, cohesives, LOCTITE(TM), superglue, fingernail glue, hand cream, and the like.

In yet another alternative, the selenium compounds of the present invention may be encapsulated in nanoparticles and/or drug delivery systems. Examples of compositions in which the selenium compounds of the present invention may be encapsulated include, but are not limited to, SWNTs, polymers, liposomes, vesicles, micelles, and the like.

In one aspect, the invention provides a method of treating or preventing growth of a species of interest through contact of a subject with a polymer matrix comprising a selenium composition of the formula R-Se-X, as described in detail herein, wherein the selenium composition comprises an inorganic or organic selenium compound, or formulation thereof, capable of generating superoxide radicals in the presence of a species of interest. The superoxide radicals generated by the inorganic or organic selenium compound inhibit or inactivate an agent of the species of interest and thereby treat or prevent growth of the species of interest in or on the subject.

In another aspect, the invention provides a method of treating or preventing the development or transmission of a species of interest in or on a subject through the use of a polymer matrix comprising a selenium composition of the formula R-Se-X, as described in detail herein, wherein the selenium composition comprises an inorganic or organic selenium compound, or formulation thereof, capable of generating superoxide radicals in the presence of a species of interest. The method involves providing the biocidal composition capable of generating superoxide radicals in the presence of an infectious agent, and applying an effective amount of the biocidal composition to the subject. The superoxide radicals generated by the inorganic or organic selenium compound inhibit or inactivate the species of interest and thereby treat or prevent the growth or transmission of the species of interest in the subject.

In a further aspect, the invention provides polymer matrices having, on at least one surface thereof, an effective amount of an inorganic or organic selenium compound, or formulation thereof, capable of generating superoxide radicals in the presence of a species of interest or reduced thiol compound or other electron donating group. The inorganic or organic selenium compound may be covalently or non-covalently associated with the composition, and an effective amount of the inorganic or organic selenium compound, or formulation thereof, is retained on or available to a surface of the composition when the composition is in contact with a subject.

In particularly useful embodiments of the biocidal compositions of the invention, the effective amount of the inorganic or organic selenium compound, or formulation thereof, that is retained on or available to a surface of the composition when such composition is in contact with the subject is sufficient to inhibit or inactivate an agent of infectious disease or other undesired cell(s).

In other embodiments, the inorganic or organic selenium compound, or formulation thereof, does not comprise a thiol group or a thiol-containing compound. In particular embodiments, the inorganic or organic selenium compound, or formulation thereof, does not comprise glutathione.

In further particular embodiments, the inorganic or organic selenium compound, or formulation thereof, is non-covalently associated with the biocidal composition. In other embodiments, the inorganic or organic selenium compound, or formulation thereof, is covalently associated with the biocidal composition.

In particularly useful embodiments, at least about half of the elemental selenium from the inorganic or organic selenium compound, or formulation thereof, exists in an active state that is capable of generating superoxide radicals in the biocidal composition. In further particular embodiments, the biocidal composition has between about 0.01 µg and about 100 µg of elemental selenium per square centimeter of surface area. In further useful embodiments, the biocidal composition has between about 1 [mu]g and about 10 [mu]g of elemental selenium per square centimeter of surface area. In yet further particular embodiments, the biocidal composition has between about 5 µg and about 6 µg of elemental selenium per square centimeter of surface area.

In general, the invention provides inorganic and organic selenium compounds, formulations thereof, and associated selenium-carrying biocidal compositions and methods for use thereof in treating or preventing an agent of infectious disease such as a bacteria, a virus, a fungus, or a protozoa, or treating or preventing growth of undesired cells. The details of the proposed chemical mechanism of superoxide formation by selenium, and selenium's proposed involvement in toxicity and carcinostatic activity in vivo, has been reviewed by Spallholz ((1994) Free Radical Biology & Medicine 17: 45-64), the contents of which is incorporated by reference herein in its entirety. In accordance with the present invention, any aliphatic or aromatic residue can be utilized as the R group in the selenium compound R-Se-X, as long as the selenium compound is capable of generating superoxide upon reacting with the sulfhydryl groups.

The method of the present invention can be used for the treatment of various surfaces and solutions to inhibit the growth of microorganisms and other undesired cells thereon. Thus, as a further aspect of the present invention there is provided a method for inhibiting the growth of microorganisms on a surface, or in a liquid, the method comprising treating the surface or liquid with a biocidal composition as hereinbefore defined. The biocidal compositions of the present invention can be used in any condition in which microorganisms or other undesired cells grow and cause problems such as, for example, but not by way of limitation, in aqueous environments including but not limited to, cooling water systems, paper mill liquors, metal working fluids, geological drilling lubricants, polymer emulsions, emulsion paints, and the like. The biocidal compositions of the present invention can also be used to impregnate solid materials or can be coated onto the surfaces thereof directly or incorporated into a paint, varnish or lacquer that is then disposed on the surface of the solid material. The biocidal compositions of the present invention may also be used to inhibit the growth of microorganisms in agricultural and horticultural environments, such as but not limited to, living plants, seeds, and the like.

Examples of applications of the biocidal compositions of use in the present invention are in particular, but not limited to, surface coatings, protective paints, and other coatings in the following: roofing, basements, walls, facades, greenhouses, sun protection, garden fencing, wood protection, tent roof material, fabrics; sanitary: public conveniences, bathrooms, shower curtains, toilet items, swimming pools, saunas, jointing, sealing compounds; requisites for daily life, machines, kitchen, kitchen items, sponge pads, recreational products for children, packaging for food or drink, milk processing, drinking water systems, cosmetics; machine parts: air conditioning systems, ion exchangers, process water, solar-powered units, heat exchangers, bioreactors, membranes; medical technology: contact lenses, bandages, diapers, membranes, implants; consumer articles: automobile seats, clothing (socks, sports clothing, and the like), hospital equipment, doorhandles, telephone handsets, public conveyances, animal cages, cash registers, carpeting, wallpapers; boat hulls, docks, buoys, drilling platforms, ballast water tanks construction; and the like.

Other specific uses for which the biocidal compositions of the present invention are suitable include, but are not limited to, soap, shampoo, skin care medicaments, paint, or incorporated into or onto plastic or a woven or non-woven fibers, when formulated to contain the requisite Se compound. The biocidal compositions of the present invention are particularly useful in the form of paints, including but not limited to, indoor and outdoor household paints; industrial and commercial paints; marine paints for use, for example, on ship's hulls; and for use as an "in-can" preservative during storage of any of the paints listed above and prior to use of such paint. The paint composition containing the biocidal composition of the present invention may be used as a paint for natural or synthetic materials, for example wood, paper, metals, textiles and plastics.

Another significant use for the biocidal compositions of the present invention is as a latex tile adhesive typically containing, for example, in addition to the biocidal component, a latex emulsion, an optional rosin emulsion, an optional plasticizer, an optional antioxidant, and an optional pigment or filler (such as calcium carbonate). Yet another significant use for the biocidal composition of the present invention is as a latex caulk, typically containing, in addition to the antimicrobial component, an acrylic latex, a nonionic surfactant, a dispersant, an optional plasticizer, and an optional pigment or filler (such as calcium carbonate). In addition, the biocidal compositions of the present invention may likewise be used as an additive for surface coatings in the maritime sector, in particular for eliminating larval barnacles on boat hulls, and generally as an additive in antifouling paints, particularly in sea water in which salt is present, and as a biofouling inhibitor in cooling circuits.

The present invention also provides for the use of the biocidal compositions of the present invention in producing hygiene products or items for medical technology. Examples of hygiene products of this type include, but are not limited to, toothbrushes, toilet seats, combs, bandages, packaging materials, as well as any article which can come into contact with many people, for example but not by way of limitation, telephone handsets, stair rails, door handles, window catches, and also grab straps and grab handles in public conveyances. Examples of items for medical technology include, but are not limited to, bandages, catheters, tubing, protective or backing films, surgical instruments, and the like.

The method of the present invention is useful, in any of the variety of applications described herein, as disinfectants and preservatives, in a liquid or spreadable solid form, alone or in combination with an inert carrier such as water, liquid hydrocarbons, ethanol, isopropanol, or the like. They can be employed using conventional procedures to control bacteria and fungi in various substrates, and can be applied to bacterial or fungal organisms or their substrates in an antimicrobial amount by conventional procedures such as spraying, dipping, drenching, impregnation, and the like.

The selenium compounds that are used in the present invention are soluble in many polar solvents, although the solubility is dependent on the nature of the R group(s) of the selenium compound. However, many of the selenium compounds are soluble in water, alcohols, ethers, ketones and other polar solvents or mixtures thereof.

The biocidal composition may be incorporated into the medium to be protected using any suitable mixing technique. It will be appreciated that the quantity of the biocidal composition required will be dependent on various factors such as the medium to be protected, the micro-organisms against which protection is desired, and the extent of protection required.

If the biocidal composition of the present invention is being used to preserve a solid substrate, the composition may be applied directly to the substrate or may be incorporated into a coating composition such as but not limited to a paint, varnish or lacquer which is then applied to the substrate. Alternatively, the solid material may be impregnated with the biocidal composition of the present invention.

### EXAMPLES AND TECHNIQUES

### Technique 1 : Method for the Fluorometric Micro-determination of Selenium

This example describes the fluorescence-based assay used to measure the total amount of selenium in a sample.

Standard Selenium Solution: a stock Se solution (1 mg Se/ml) was prepared by dissolving 109.5 mg sodium selenite (MW: 172.9) in 50 ml analytical-grade water. From this stock solution 1 [mu]g Se/ml and other standard Se solutions were prepared by dilution.

Digestion Mixture: 225 ml of concentrated sulfuric acid was slowly added to 225 ml analytical-grade water, and 300 ml of 70-72% perchloric acid was added.

Diaminonaphthalene Solution: Diaminonaphthalene (DAN) solution was prepared just prior to use by dissolving 75 mg of 2,3-diaminonaphthalene in 75 ml DAN acid solution (prepared by slowly adding 150 ml sulfuric acid to 850 ml analytical-grade water prior). The resulting solution was transferred to a separatory funnel, and the DAN solution was washed with HPLC grade cyclohexane with vigorous shaking. After allowing time for separation of the phases, the upper layer was tested by fluorescence meter until a reading of less than or equal to 0.2 was reached. The lower layer was then collected for immediate use.

Disodium EDTA Solution: 0.008 M EDTA solution was prepared by dissolving 2.98 g of disodium ethylenediaminetetraacetic acid hydrate in 1 L of analytical-grade water.

Digest Sample: A desired amount of sample was added to a 25 x 200 mm test tube, followed by 3 ml of digestion mixture and 2-3 glass beads (4 mm). The samples were digested over a burner under a perchloric acid hood. 4 ml EDTA was added to each sample. At this time all samples were, and should be, colorless (otherwise the digestion is incomplete). The pH was then adjusted to 2.0 - 2.2 using ammonium hvdroxide or sulfuric acid.

Fluorescence test: 4 ml of freshly prepared DAN reagent was added to each sample. The resulting solution was mixed well, and incubated in a water bath at 50 [deg.]C for 30 minutes. After incubation, the tubes were cooled for 5 min, and then 4 ml of cyclohexane was added and the tube mixed well. The upper layer (4,5-benzopiazselenol complex) was then quantitated by fluorescence meter with the excitation measured at 363 nm and the emission measured at 525 nm.

Selenium content: The standard solution was used to build a selenium content curve over fluorescence readings. Then based on the sample's reading, the corresponding selenium content of the standard curve could be extrapolated.

### Technique 2: Chemiluminescent (CL) assay

This example describes the ehemiluminescence-based assay used to measure the amount of reactive selenium available in a sample for thiol-dependent superoxide formation.

The control chemiluminescent (CL) assay cocktail without substrates or GSH was made using a 0.05 M sodium phosphate buffer (pH 7.0) and 20 [mu]L lucigenin/ml from a stock solution of 1.0 mg/ml lucigenin in distilled water. The assay cocktail with thiol contained 1.0 mg GSH/ml. To 600 [mu]L test aliquots of the control or thiol containing assay cocktail was added L-selenomethionine or L-Se-methyl-selenocysteine at 2.0 mg/ml, and other substrate concentrations were made by dilution with buffer containing lucigenin. In like manner, D, L-selenoethionine was added at 4.0 mg/ml to the CL cocktail. To the control or substrate-containing CL cocktails in the luminometer was added methioninase containing 0.5 U of enzyme activity or graded units of methioninase activity. Methioninase was prepared by adding 1.0 or 2.0 ml of distilled water to 10 U vials of commercial freeze-dried enzyme. The methioninase was reported by Waco technical services to contain no reducing thiol preservative. The enzyme was added in 0.1 ml increments from a 1.0 cm<3> syringe or up to 30 [mu]l from an Eppendorf pipette directly to the chemiluminescent tube in a Los Alamos Diagnostics Model 535 luminometer containing 600 [mu]l of the pH 7.0 cocktail. The CL tube contents was held at 36.8<0>C by an attached LKB 2209 multitemp recirculating water bath. Chemiluminesce (CL) data was recorded in 30-s integrated units over a period of up to 20 min. There was a 3-s instrumental delay between integrations. Additional details of this assay, including the quenching of chemiluminescence generated by methylselenol from reduced methylseleninic acid and dimethyldiselenide by superoxide dismutase, has been previously reported. This CL assay is quantitative (correlation coefficient, r % 0:99; P < 0:001) in generating CL for small amounts of redox cycling methylselenol. A standard curve for methylselenol (CH3SeH) produced CL (relative CL units vs. selenium concentration) from the reduction of dimethyldiselenide by GSH when added directly to the CL cocktail, can be used to facilitate quantitation of the experimental samples.

### Technique 3: Selenium Content and Superoxide Activity Analysis for Bandage Coating

To 150 g of a standard sample of a non-latex cohesive formulation (Andover Coated Products, Inc., Salisbury, MA), 0.9g of the organoselenium compound (Selenocyantoacetoxy) butoxyethyl methacrylate (SCABEM) was added drop-wise with mixing, resulting in a cohesive solution having 0.00126 gram Se/gram soln. (0.9g / 150 g non-latex cohesive = 0.006g SCABEM/g ctg; Se is 21% by weight of SCABEM = 0.006g X 0.21 = 0.00126 gram Se/gram soln.). A portion (3.43g) of this sample was drawn onto a composite cohesive elastic bandage (Andover Coated, Inc., Salisbury, MA), air dried for 5 min and then flash dried for 2 min at 100°C. From this composite drawdown, a 10 cm by 30 cm section was cut and then further cut into 1 cm squares for testing. In theory, 3.43 g of this SCABEM-containing solution distributed evenly across the 300 cm² sample should result in 0.0000144 g Se/cm² (i.e., 1.44 x 10⁻⁵ g Se/ cm² = 3.43 g soln./ 300 cm² = 0.011 g soln./ cm² (i.e., 0.011 g soln. Se/ 1 cm² sample) and 0.011 g soln/ cm² x 0.00126 g Se/ g soln. = 0.0000144 g Se/cm²). The resulting squares were tested, using the standard methodology described above, in both fluorescence and chemiluminescence assays. The fluorescence assay provides a measure of the total selenium deposited on the test square, while the chemiluminescence assay provides a measure of the total reactive selenium available to provide for thiol-dependent catalytic superoxide formation. The results are shown in Table I.

**Table I: Selenium Activity and Content Analysis for Bandage Coating**

| Sample # | | Weight (mg) | | Chemiluminescence assay | | | Fluorescence assay | |
|---|---|---|---|---|---|---|---|---|
| | | Counts after 5 minutes | Counts/weight | reading after dilution | Se content (µg) after dilution | dilution folds | total Se (µg) | Se content (µg)/weight (mg) |
| 1 | 40 | 567 | 14.18 | 12.5 | 6.93 | 8 | 55.4 | 1.39 |
| 3 | 33 | 989 | 29.97 | 15.1 | 8.65 | 8 | 69.2 | 2.09 |
| 4 | 37 | 922 | 24.92 | 14.4 | 8.19 | 8 | 65.52 | 1.77 |
| 5 | 46 | 605 | 13.15 | 17 | 9.9 | 8 | 79.2 | 1.72 |
| 6 | 44 | 005 | 22.84 | 15.8 | 9.11 | 8 | 72.9 | 1.66 |
| 7 | 45 | 782 | 17.38 | 10.4 | 5.6 | 8 | 44.8 | 0.99 |
| 8 | 44 | 825 | 18.75 | 16.9 | 9.84 | 8 | 78.7 | 1.79 |
| 9 | 40 | 819 | 20.48 | 12.1 | 6.67 | 8 | 53.36 | 1.33 |
| 10 | 42 | 869 | 20.69 | 14.3 | 8.12 | 8 | 64.96 | 1.55 |

| | | | | | | | | |
|---|---|---|---|---|---|---|---|---|
| Note: Chemiluminescence assay is to test selenium activity, while Fluorescence assay is to acquire total selenium content. All of these samples were cut off as 1 square centimeter from the bandage. | | | | | | | | |

The total amount of Se in the 12 random samples was 2117.24 µg from the fluorescence data shown in Table I (column labeled "Total Se (µg)"). This was divided by 12 samples = 176.44 µg Se/sample, and since each sample holds approximately 11 mg of the SCABEM solution coating, this is equal to 16.04 µg Se/mg SCABEM solution coating.

The total amount of luminescence (counts) afforded by the 12 random samples highlighted in the column labeled "Counts after 5 minutes" above is 12,323 (counts). This was divided by 12 samples, each hosting approximately 11 mg of coating to give 93.4 counts/mg coating. This test has been standardized such that 10 counts are the equivalent of 1 µg of Se, so the equivalent of 9.34 µg of Se/mg coating has been detected. Since the Non-Latex cohesive elastic white formulation contains a measure of optical brightener, and an average of 20 counts was detected from the chemiluminescent analysis of the blank, the actual amount of luminescence detected resulting from superoxide formation is the equivalent of 9.34 µg O₂⁻ - 2 µg O₂⁻ = 7.34 µg O₂⁻. These data have shown that 7.34 µg O₂⁻/mg coating of the total 16.0 µg Se/cm² detected in the sample is responsible for the formation of superoxide. 16.0 µg Se Total x 100% = 45.76% of Selenium is present in a reactive form capable of generating superoxide in the presence of thiol compound.

This means that the mixing of a specifically chosen organoselenium compound affords ∼46% of the antimicrobial compound at the bandage/skin interface where it produces superoxide in sufficient quantity to kill bacteria and/or other microbes.

The ability of bacteria to migrate from an untreated bottom tape to a top tape when the tapes are stacked one on top of the other and allowed to set on a bacterial lawn for 24 hours was also tested.

**TABLE II. Bacterial Migration on Stacked Tapes**

| Untreated | Treated |
|---|---|
| 13.334 | 10,100,000 |

Table II demonstrates the difference in bacterial colonies of Pseudomonas aeruginosa between those found on the bottom tape minus those found on the top tape. Thus, a larger number indicates that the top tape is resisting the migration of the bacteria. In the untreated experiment, neither tape contained a selenium coating, while in the treated experiment, only the top tape contained a selenium coating. Each number in Table II is the average of three experiments.

Thus, the results in Table II demonstrate that a selenium coating markedly inhibits the ability of bacteria to migrate onto a selenium coated tape.

### Technique 4: Solution-Based Assay for Antimicrobial Activity

Unlike other antibiotics which require metabolism of the bacteria for activity, the unique mechanism of action of the selenium technology allows for effective killing of bacteria even at 4°C. This means that a refrigerated selenium-coated surface or selenium-containing product would still be effective in killing of bacteria, which come into contact with the surface. Tests demonstrating this ability were carried out using E. coli in liquid media (LB). These tests utilized E. coli which expressed a specific antigen (*Yersinia pestis* F1 antigen) to which a selenium-coated peptide would bind. The purpose of the F1 antigen and corresponding peptide was to provide for a means of localization of the covalent seleno-peptide to the bacteria. A similar localization would exist for bacteria coming into contact with a selenium-coated surface or product

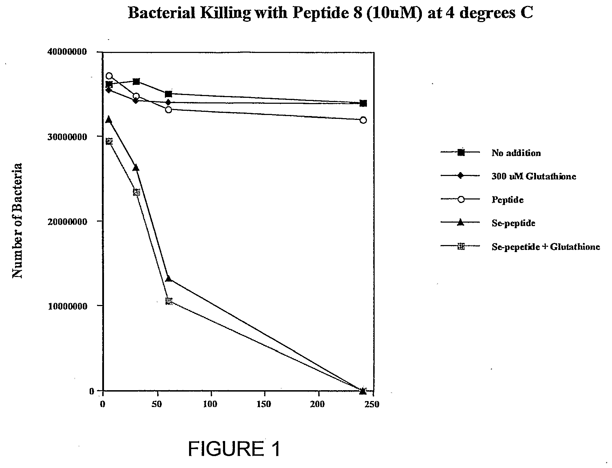

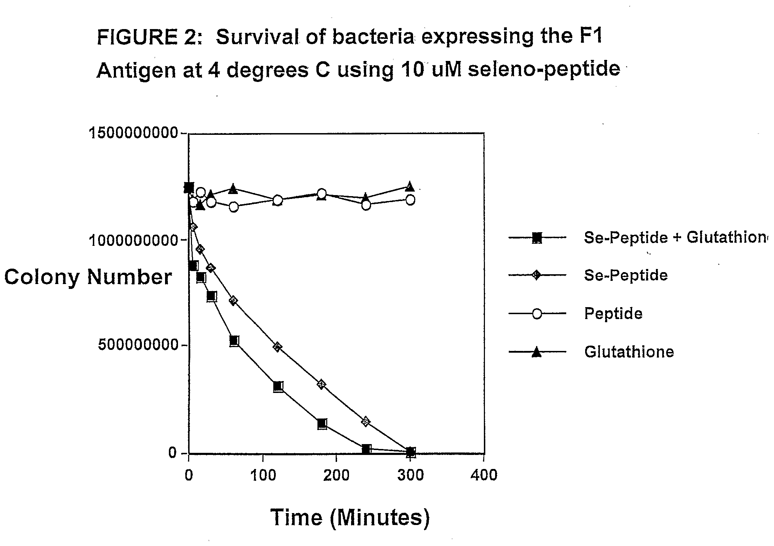

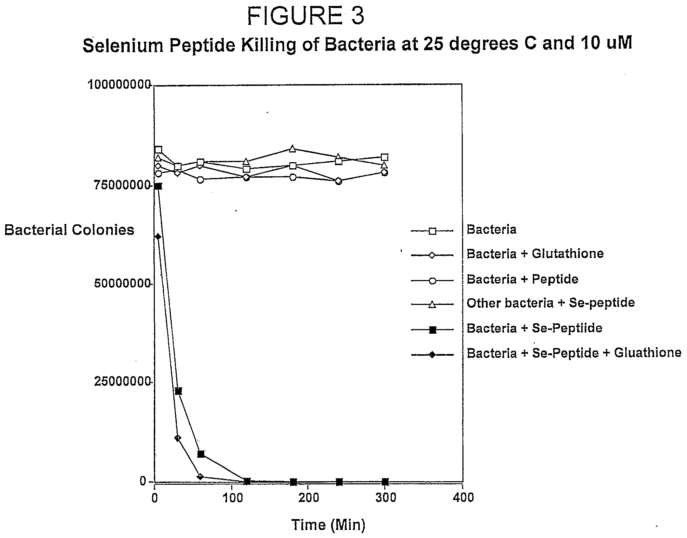

The bacteria were grown overnight at 37°C to an optical density 600 nm (OD₆₀₀) of 1. This solution was then diluted to an optical density of 0.3 (10⁸ bacteria per ml) at 600 nm (OD₆₀₀), and cells were collected by centrifugation and washed twice with PBS. The cells were resuspended in H₂O, and aliquots equivalent to 1 ml of culture at 0.3 A₆₀₀ (∼10⁸ cells) were transferred to 1.5 ml microcentrifuge tubes. The tubes were placed on ice for at least 15 minutes before starting the experiments, and they were kept on ice during the course of experiments. Seleno-peptide and peptide without selenium were added to bacterial suspension at a concentration of 10 µM with and without glutathione at 300µM final concentration, and the peptides mixtures were incubated along with the control solutions (also with and without glutathione at 300µM final concentration) on ice and were kept mixed by the bubbling of air. At different time intervals, an aliquot was removed from each mixture and a 1:100 serial dilutions was made; the diluted aliquots were then plated on LB plates (10 g/L Bacto-Tryptone, 5 g/L yeast extract, 15 g/L Bacto Agar, 5 g/L NaCl, and 100 [my]l_10N NaOH) for E. *coli XLI-*Blue parent strain, and LB (10 g/L Bacto- Tryptone, 5 g/L yeast extract, 15 g/L Bacto Agar, 5 g/L NaCl, and 100µL 10N NaOH) supplemented with Carbenicillin (10[mu]g/ml) to enumerate viable bacteria. The same culture dilution is used for all plates so that a comparison could be made between colony counts. The plates were incubated at 37°C for at least 24 hours, and colony forming units were counted. FIGS. 1-3 show the results of these experiments. With the selenium-conjugated peptide, complete killing of bacteria was recorded in almost 4 hours, while control treatments resulted in complete E. coli viability. Identical tests were performed with *E*. *coli* not expressing the F1 antigen. In such cases none of the selenium-conjugated peptides caused killing, confirming the necessity of the binding the seleno-peptide to *E*. *coli* in order to achieve anti-bacterial stasis.

FIGS. 1 -3 demonstrate that a low concentration of a specific selenopeptide (10 µM) can kill over 8 logs of bacteria in 60 minutes at 25°C and over 8 logs of bacteria in 250 minutes at 4°C.

This rapid response is very specific: only bacteria expressing the antigen were killed, whereas bacteria without this binding site were not killed. In addition, the killing ability at 4°C demonstrates the ability of the biocidal compositions of the present invention to be effective at a refrigerated temperature. Such ability would be advantageous in areas such as but not limited to, tissue preservation and blood banking.

### Technique 5: Dose Response of Known Concentrations of Selenofolate and the Amount of Chemiluminescence Generated

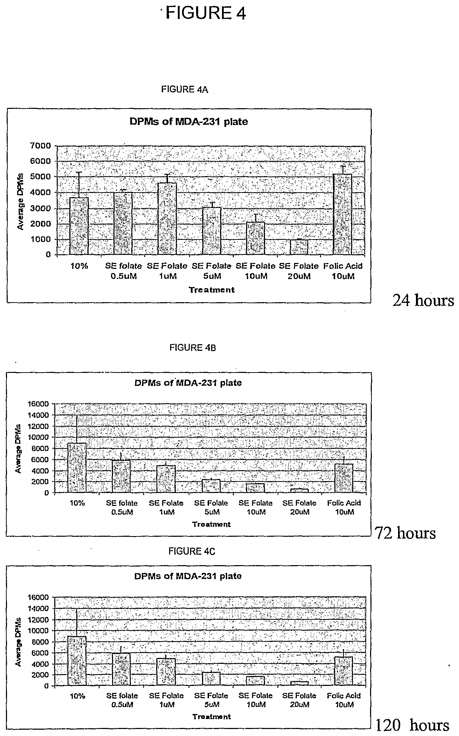

Two chemiluminescence (CL) trials were run at different selenofolate concentrations. The first trial is shown in Table IHA. Pearson's r for these CL values vs. concentration (volumes) was 0.926. The second trial was a 2:1 dilution of the concentrations used in the first trial and is shown in Table HIB. Again, CL vs. concentration of Se had a Pearson's, r = 0.982, which is slightly better than the first trial due to the lesser counts and better spread of concentration. If these samples were analyzed for Se by fluorometry, the concentration of Se would correlate with the CL, and the killing profile for selenofolate (FIGS. 4A-4C). Thus, cell killing, CL and selenium concentration are intricately entwined owing to the generation of superoxide and other reactive oxygen species (ROS).

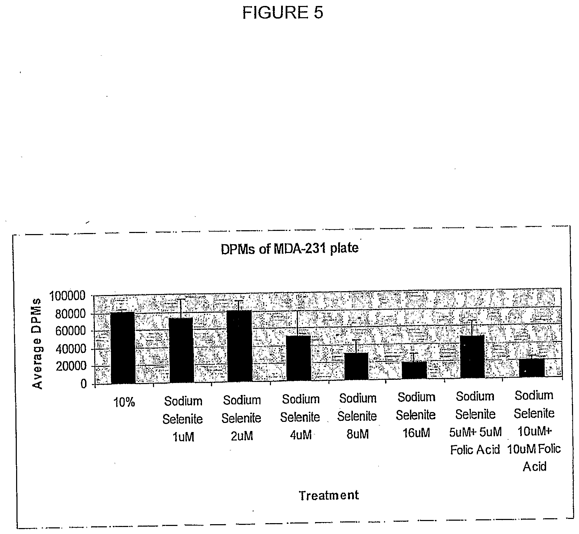

In comparison, FIG. 4A-4C illustrates both selenofolate concentration and time effects on breast cancer arrest in MDA-231 cells at 24 hours, 72 hours and 120 hours. This result can also be compared to FIG. 5, which demonstrates a dose response of MDA-231 breast cancer cells to selenite; when compared to selenofolate, cells exposed to selenite are somewhat resistant to this particular ROS.

FIG. 4 illustrates a dose response study for the treatment of breast cancer cells (MDA-231) in culture in 10% fetal calf serum with selenofolate. These cells were treated 24 hours after plating the cells. The treatment was then allowed to go for 24 hours (4A); 72 hours (4B); or 120 hours (4°C).

FIG. 5 illustrates a dose response study for the treatment of breast cancer cells (MDA-231) in culture in 10% fetal calf serum with sodium selenite. These cells were treated 24 hours after plating the cells. The treatment was then allowed to go for 24 hours.

**TABLE IIIA. Chemiluminescence at Different Selenofolate Concentrations**

| Amount of Addition of Stock Solution (µl) added to 1µlselenofolate | Total Counts |
|---|---|
| 0.0 | 0 |
| 1.25 | 3.023 |
| 2.5 | 5.434 |
| 5.0 | 13.039 |
| 10.0 | 14.459 |

**TABLE IIIB. Chemiluminescence at Different Selenofolate Concentrations**

| Amount of Addition of Stock Solution (µl) added to 1µlselenofolate | Total Counts |
|---|---|
| 0.0 | 0 |
| 2.5 | 3.023 |
| 5.0 | 3.206 |
| 20.0 | 8.142 |
| 40.0 | 12.836 |

### Example 1: Prevention of Bacterial Colonization of Contact Lens

Contact lenses are worn by approximately 90 million people worldwide. Early contact lenses were required to be taken out of the eye daily for removal of accumulated protein and lipids discharged by the eye and for sterilization. In addition, poor oxygenation of the cornea, caused by the relatively low oxygen transmissibility of early conventional hydrogel lenses, also limited wearing time. Whereas the great majority of contact lens wearers still remove their lenses daily, the recent arrival of silicone hydrogel lenses, which provide greater oxygen transfer to the cornea, allow the
longer term 30-day wear that is now permitted by the FDA. However, despite the improved corneal physiology provided by the new highly oxygen permeable 30-day lenses, bacteria that cause acute red eye or even corneal ulceration are still a clinical concern.

When a contact lens is placed on the eye, the surface of the contact lens is colonized by bacteria more than 50% of the time. The role that this colonization plays in the success of contact lens wear has been a topic of interest for decades. Studies that look at this issue generally fall into 2 categories: studies that have an interest in the characteristics of the bacterial colonization in asymptomatic patients and studies that looked at bacterial colonization in patients with acute red
eye or infection. A number of earlier studies used culturing techniques or scanning electron microscopy (SEM) to look at bacteria that are loosely and/or tightly bound to contact lenses taken from asymptomatic patients. There seems to be general agreement across studies to date that the make up and density of bacterial colonization do not change with changes in the contact lens material. How the number of bacteria varies over time and wearing schedule remains less clear.
Inflammatory reactions or infections have been reported to be associated with bacterial colonization of the soft contact lens surface, particularly when the lenses are worn for extended time periods. Whereas microbial keratitis does not seem to be exclusively associated with bacteria of a particular gram stain, gram-negative bacteria seem to be associated with contact lens-induced acute red eye, and gram-positive bacteria are associated with contact lens induced peripheral ulcers. Baleriola-Lucas et al. (1991) found a clear association between acute red eye responses in extended wear and gram-negative bacterial contamination, with *Serratia marcescens* and *S*. *liquefaciens* dominating. They felt that their results suggest that there may be an association between bacterial colonization of hydrogel contact lenses and acute adverse responses.

Given the direct and indirect evidence that bacterial colonization of contact lenses is closely associated with acute red eye and corneal infections, a material or coating that inhibits bacterial colonization should substantially improve the safety of contact lens wear. One candidate to serve this role is a covalent coating of selenium (Se). To increase the safety of extended wear contact lenses, researchers have sought biomaterials that reduce the adverse consequences caused by bacterial colonization. No material has been reported to date that prevents bacterial colonization in extended wear lenses and, at the same time, is compatible with the cornea. The present invention demonstrates, for the first time, the covalent attachment of Se to the surface of extended wear silicone hydrogel lenses, Se's bactericidal efficacy against bacterial colonization, and its compatibility with the cornea of the rabbit. The surface of the lens modified with Se is demonstrated to be catalytically active *in vitro,* producing superoxide as detected by lucigenin chemiluminescence. The Se-modified hydrogel lens is also shown by SEM to prevent *in vitro* colonization by *Pseudomonas aeruginosa,* which otherwise occurred on uncoated (control) lenses. Furthermore, the coated Se lens caused no detectable changes in the cornea of New Zealand White Rabbits fitted with Se and control lenses for periods up to 60 days. The results are suggestive for a wide application of active Se-coated surfaces for the prevention of bacterial colonization on silicone hydrogel contact lenses and catheters, stents, glaucoma valves, heart valves, and the like.

### Materials and Methods:

Attaching Se to the Contact Lenses: PureVision (balafilcon A) contact lenses (Bausch & Lomb, Inc, Rochester, NY) were used, and the contact lenses were covalently coated with Se as described herein above. The coating process results in the covalent attachment of an organo-Se molecule directly to the surface of the contact lens. Each lens came with a certificate, supplied by
Selenium Technologies Inc (Lubbock, TX), stating the amount of active Se on each lens. The amount of Se covalently attached to the contact lenses was determined by the fluorometric method described in Example 1. Briefly, a contact lens from the same batch was placed in a perchloric/sulfuric acid solution in a Pyrex test tube and was digested over a Fisher burner in a perchloric acid hood. The solution was neutralized and EDTA was added followed by the addition of diaminonaphthalene. The resultant selenodiaminonaphthalene complex was extracted into cyclohexane. The cyclohexane containing the selenodiaminonaphthalene complex was quantified by a Perkin-Elmer Model 650 Y 40 fluorometer (Wellesley, MA) at excitation 363 nm per emission 525 nm (see Example 1). From this method, it was evident that each treated lens was coated with 8 µg of Se per lens. The amount of active Se determines the amount of superoxide that can be generated by the Se-coated lens in a specific amount of time. Selenium-coated lenses were marked with a small dot placed on the anterior surface using a Sharpie permanent marker (Sanford, Inc, Bellwood, IL).

Lenses were evaluated for Se activity after up to 2 months of wear in the rabbit eye. This was done to determine if the Se was still attached to the lens and if the Se activity was decreased by protein or lipid deposition. Selenium activity was measured by chemiluminescence (Spallholz et al. (2001) Nutr Cancer, 40:34-41). Chemiluminescence was measured, using lucigenin as the detector of superoxide, by adding the contact lens to a tube containing 1 mL of a sodium borate (0.05 M) buffer, lucigenin (20 µg/mL), and reduced glutathione (4 mg/mL) (Sigma Chemical) solution. The luminescence from the solution was then measured by counting photons that are released in the interaction between superoxide radicals with lucigenin. Photons were counted during fifteen 30-second intervals using a Los Alamos Diagnostics (Model 535) chemiluminometer maintained at 36°C. In addition, a small sample of Se-coated contact lenses that were not used in further experiments was evaluated to measure activity soon after the Se coating was applied.

Testing of the Se-coated Contact Lenses in a Rabbit Eye: New Zealand White Rabbits (body weight 3.5-4.5 kg; male) were used in this study to provide larger eyes. All rabbits were treated according to the ARVO Resolution on the Humane Use of Animals in Ophthalmic and Vision Research. All animals were healthy and free from observable ocular disease. The nictitating membranes were left intact. All animals were used after a minimum 2-week recovery and maintained in individual cages at a constant room temperature (25°C) with a relative humidity of 30-70%. A standard 12-hour light-dark cycle was maintained. 15 rabbits were used for the experiment. Attempts were made to keep the pair of contact lenses (1 Se-coated, 1 control) in a minimum of 10 rabbits' eyes continuously for 2 weeks. Lenses were then removed and replaced with a new pair (1 Se-coated, 1 control) for an additional 2 weeks. Rabbits were checked twice per
day to monitor the ocular reaction to the contact lenses and to be certain that the contact lenses were in the eyes. Any lenses that fell out were rinsed and disinfected with Opti-Free multipurpose solution (Alcon Laboratories, Inc, Fort Worth, TX) and returned to the rabbit's eye. Three rabbits that continually lost their contact lenses were dropped from the study. The contact lenses taken from the rabbits that successfully wore the lenses were divided into the experiments in the study.

### Monitoring of Rabbit Corneal Toxicity:

Slit-Lamp Exam: Slit-lamp observations were performed before the contact lenses are placed on the eyes and at weekly intervals after insertion. Slit-lamp findings included staining, hyperemia, neovascularization, edema, infiltrates, and iritis. After FDA 510(k) guidelines, slit-lamp findings were recorded on a 0-4 scale with 0 indicating none; 1, trace; 2, mild; 3, moderate; and
4, severe. The person performing the slit-lamp observations was not masked as to which eye contained the treated lens.

Pachymetry: Pachymetry was performed before lens insertion and after 2 weeks with a Corneo Gauge Plus pachymeter (Sonogage, Inc, Cleveland, OH) to look for corneal swelling or tissue loss. The corneas were measured using 2 of the measurement modes available on this pachymeter: RK yields full corneal thickness data and LK yields epithelial thickness data. With both of these modes, pachymetry was performed 3 times at each of 5 locations on the rabbit corneas. The 5 locations included a central location and 4 locations that were 3-mm superior, inferior, lateral, and nasal from the center of the cornea. The 3-mm distance was estimated by taking a pachymetry measurement halfway between the center of the cornea and the limbus, because the rabbit cornea is about 12 mm in diameter (Brem (1975) J Exp Med, 141:427-439).

SEM: At the completion of 2 months, the animals were sacrificed and half of the cornea was fixed in 2% glutaraldehyde in 0.1 M Phosphate pH 7.2 buffer followed by a 1% OsO₄ postfix for SEM studies after the method of Hazlett (Prog Histochem Cytochem (1993) 25:1-60).
The specimens were examined for changes in the morphology of the cornea. The corneas were scored on a 0-4 scale with 0 indicating no change; 1, slight ruffling of cells; 2, gaps between cells; 3, mild cellular toxicity; and 4, extensive cellular toxicity.

Histopathology: Histopathology was performed on the other half of each cornea. This portion of the study was carried out in the Department of Pathology, University of Wisconsin, Madison, School of Veterinary Medicine. Corneas were evaluated for signs of possible toxicity due to the Se attached to the contact lens. The corneas were scored on a 0-4 scale, with 0 indicating normal; 1, abnormal cells present; 2, intermittent areas of cellular death; 3, large sections of cellular death; and 4, extensive to total death of the epithelial cells.

### Monitoring of the Contact Lens Surface

SEM of Bacterial Colonization: In a separate experiment from the rabbit experiment, treated and control contact lenses were incubated *in vitro* at 37°C for 4 days in L-broth inoculated with P. *aeruginosa* at log-phase growth. Lenses were rinsed in distilled water and then fixed in 2% glutaraldehyde in 0.1 M Phosphate pH 7.2 followed by a 1 % OsO₄ postfix and submitted to SEM. The treated and control contact lens surfaces were examined for deposition and bacterial adhesion. Six sites were chosen with a random spin of the wheel that controlled the position of the contact lens in the microscope. The pictures chosen for this paper were representative of the average of these 6 locations.

Protein Coating of the Se-coated Contact Lenses: A subset of the contact lenses worn by the rabbits was analyzed for the amount of protein adhering to the lens surface. The lenses were removed with sterile contact lens forceps and vigorously rinsed with nonpreserved saline before the extraction procedure. The protein extraction for individual contact lenses followed the procedure utilized by Minarik and Rapp (J CLAO (1989) 15:185-188) as follows:
1. The lens was placed in 1.5 mL of distilled water containing 1 drop of urea solution (2 g/mL). The solution was stirred for 30 minutes.
2. The solution was removed.
3. The lens was placed in 1.5 mL of 1 N NaOH and spun for 30 minutes; the solution was pooled with the solution from step 2.
4. The lens was placed in 1.5 mL distilled water and 1 drop DMSO was added; spun for 30 minutes; the solution was pooled with the solution from step 3.
5. The lens was placed in 1.5 mL of 25% isopropyl alcohol; spun for 30 minutes; the solution was pooled with the solution from step 4.
6. The lens was placed in 1.5 mL of distilled water and 1 drop of urea (2 g/mL) was added; spun for 30 minutes; the solution was pooled with the solution from step 5.
7. The lens was placed in 1.5 mL of 20% acetic acid; spun for 30 minutes; the solution was pooled with the solution from step 6.

The pooled extract was analyzed for total protein using the Bio-Rad Protein Assay, a dye binding assay based on the differential color change of a dye in response to different protein concentrations.

Lipid Deposition on Se-coated Contact Lenses: Another subset of the lenses worn by the rabbits was analyzed for the amount of lipid adhering to the lens surface. The lenses were removed with sterile contact lens forceps and vigorously rinsed with nonpreserved saline before the extraction procedure. The lipid extraction for individual contact lenses followed a modification of the procedure of Bontempo and Rapp (Curr Eye Res. (1997) 16:1258-1262) as follows: lenses were incubated in a large, closed glass tube with 150 µL of hexane with continuous shaking overnight. Five microliter of the extract was then placed on a TLC plate. The plate was then developed with ethyl acetate/hexane (1:4). The density of the lipid spots were then determined with a densitometer.

### Results:

Se Activity Before and After Wear: Selenium activity was determined by chemiluminescence
before and after 2 months of wear in a rabbit eye. The lenses were found to have 2.14 ± 0.2 chemiluminescent unitsbefore wear and 1.90 ± 0.2 after wear. Thus, there was little loss of Se activity with 2 months of contact lens wear and deposition.

### Corneal Toxicity

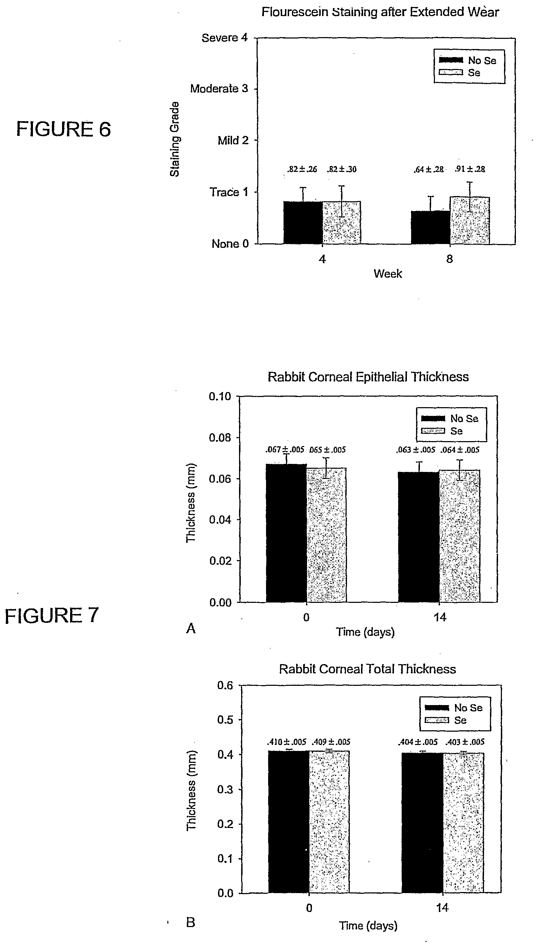

Slit-lamp Data: Whereas the weekly slit-lamp evaluations looked for fluorescein staining, hyperemia, neovascularization, edema, infiltrates, and iritis, the only clinical sign to show nonzero
data was fluorescein staining for either the control or experimental eye. Thus, FIG. 6 presents the fluorescein staining data from the observations on 11 rabbits at 4 and 8 weeks. The fluorescein staining consisted of occasional isolated punctate stains and, more frequently, randomly located light arcuate or near-linear stains. Whereas no formal analysis was run, these arcuate and linear stains seemed to occur more frequently in the feistier rabbits and, thus, might be attributable to contact lens removal trauma. As shown in FIG. 6, neither the treated nor the control lenses produced significant corneal staining with no significant difference between the control and experimental eyes.

Pachymetry Data: Pachymetry data from the 5 measured locations on both corneas of 10 rabbits were collated and averaged separately. When no site-specific trends were found, the data were recollated and averaged with data from all 5 measurement locations grouped together. FIG. 7A and B presents the epithelial and total cornea pachymetry data at time 0 and after 2 weeks of contact lens wear. Neither the epithelial nor the total corneal thickness data showed significant changes in thickness. Thus, little indication was found that the corneas that wore the treated or the
control lenses suffered any tissue loss or edema.

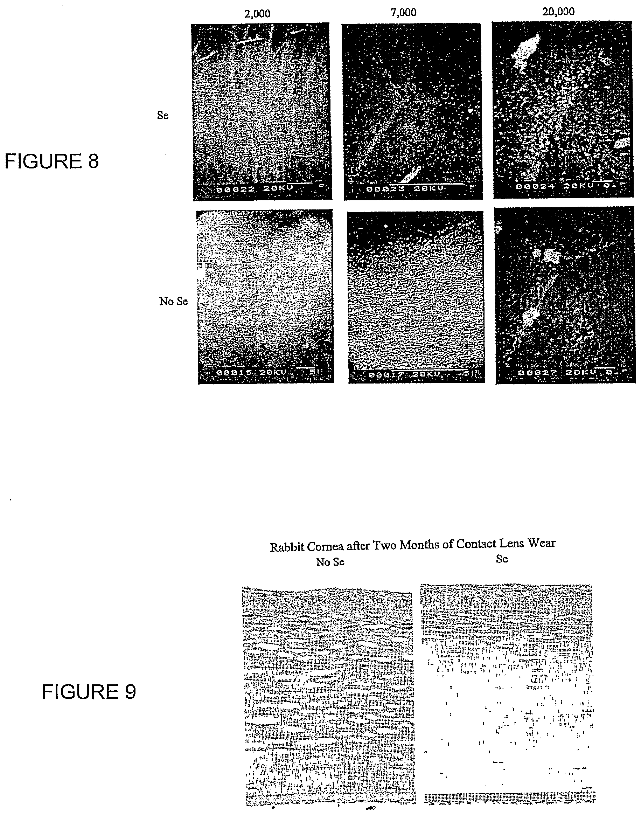

SEM Data: All electron micrographs of the anterior corneal surface were graded on the scale described above. All control and experimental corneas received a 0 score (no change). FIG. 8 shows representative sample electron micrographs that illustrate the similar appearance of corneas that wore a Se-coated lens versus an uncoated lens for 2 months. The electron micrographs show that the junctions between cells are still tight in both corneas. In addition, the microvilli are undisturbed in both corneas.

Corneal Histology Data: All cornea samples sent for histological examination also scored 0 (normal) on the histology scale described above. FIG. 9 shows representative corneal histology slides that show no difference between corneas that wore a Se-coated lens versus an uncoated lens for 2 months.

### Lens Surface

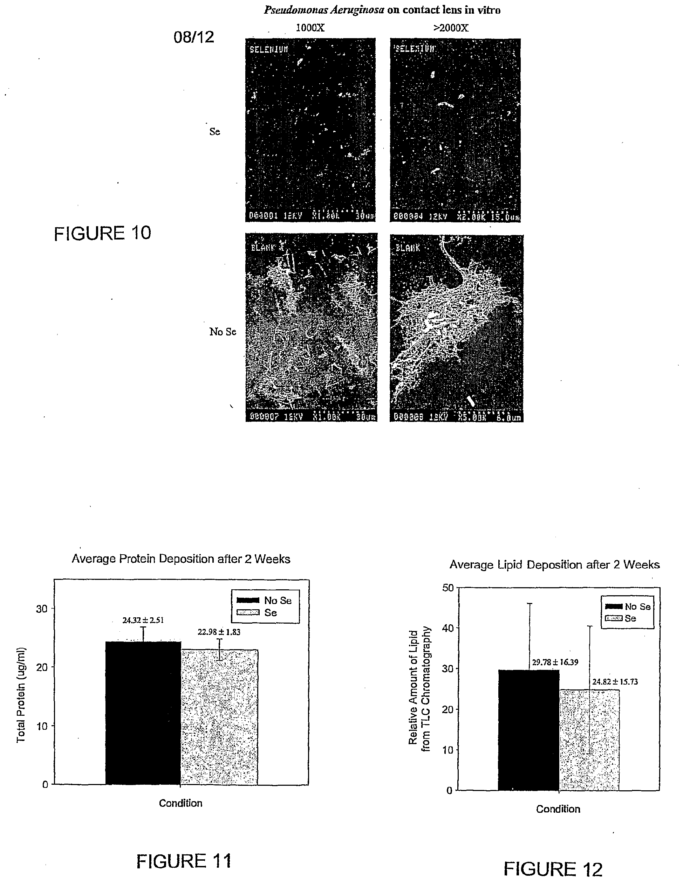

SEM: Electron micrographs (FIG. 10) show little or no bacterial colonization on the Se-coated lenses and extensive colonization on the uncoated lenses with *P. aeruginosa.* Contact lenses with the Se treatment had to be searched to find the minimal bacteria and debris illustrated in this figure, whereas the untreated lens had dense colonization over a majority of the contact lens surface.

Protein Deposition: There was no significant difference in total protein deposition (FIG. 11) between 9 Se-coated and 9 uncoated control lenses after 14 days in rabbit eyes. Whereas the treated lenses (Se) had a slightly lower mean total protein level, the SEs of the mean easily exceeded the difference in the means.

Lipid Deposition: There was no significant difference in lipid deposition (FIG. 12) between 9 Se-coated and 9 uncoated control lenses. In addition, there was considerable variability in the amount of lipid found on both the treated and control lenses resulting in large confidence intervals. Whereas the treated lenses (Se) had a slightly lower mean relative lipid level, the SEs of the mean easily exceeded the difference in the means.

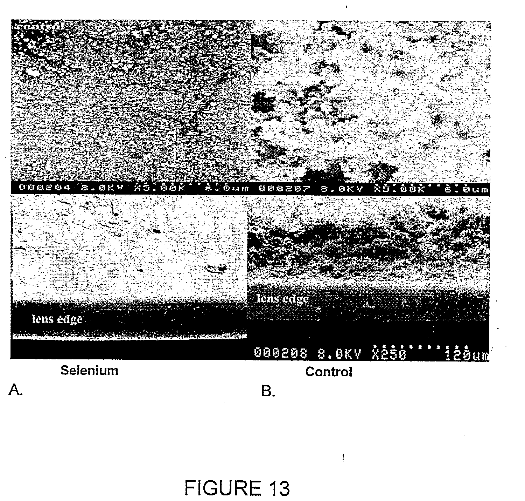

In FIG. 13, two contact lenses (one coated with selenium and the other without selenium) were each worn in a human eye for one week without removal. The contact lenses were then placed in an L-broth media and inoculated with *Staphylococcus aureus.* This bacteria was allowed to grow for 4 days. At the end of this time the lenses were removed, gently rinsed with water and processed for scanning electron microscopy. FIG. 13A shows the selenium coated lens. Only a few bacteria were found to be present. FIG. 13B is the control lens without selenium. As can be seen, a thick biofilm was present on the control lens.

### Discussion:

Historically, three of the major problems limiting the safety and duration of contact lens extended wear have been oxygen transmissibility, bacterial colonization, and protein/lipid deposition. The relatively recent introduction of silicone hydrogel materials has gone a long way toward relieving concerns about oxygen transmissibility. The present study suggests that a monomolecular coating of Se could solve the second problem. The Se-coated lenses showed a marked tendency to resist bacterial colonization *in vitro* as observed by electron microscopy. On the other hand, the data showed no reduction (or expansion) of protein or lipid deposition on the treated lenses. Finally, multiple *in vivo* tests suggest that the Se-coated lenses are safe to the rabbit cornea.

Electron micrographs of Se-coated and control contact lens surfaces show that the Se-coated lenses placed in a broth inoculated with bacteria resisted the bacterial colonization. Whereas culturing lenses removed from the rabbit eyes might have been closer to the real world use of contact lenses, it was decided to test the efficacy of the Se-coating by exposing the lenses to a high bacterial load. The bacterial load in the *in vitro* experiments is much greater than in a normal eye. In addition, there are no passive or active immune systems *in vitro* to help the Se prevent bacterial attachment like there are in a normal eye. The two immune systems have probably contributed to the marked variability seen in previous experiments that cultured contact lenses taken from patients. Of course, there is the potential that the protein and lipid deposition found in either the rabbit or the human eye could "smother" the Se coating and, thus, reduce or eliminate its bactericidal effect. This did not seem to be the case in a subset of lenses worn by the
rabbits for 2 weeks and placed in the bacterial broth. Whereas cell count studies were also initiated, electron microscopy is considered to be the optimal method for investigating bacterial colonization.

The data showed no significant effect on protein or lipid deposition on the Se-coated versus the control lenses after being worn by rabbits for 14 days. The expectation of a difference in protein deposition was based on the fact that bacteria attaches itself to a surface by secreting a protein matrix onto the surface. It was anticipated that making a contact lens surface resist bacteria might also decrease the total protein and, possibly, lipid bound to the lens surface. This seems not to be the case. On the other hand, the Se coating also did not adversely affect the deposition characteristics of the silicone hydrogel material in the study lenses. Senchyna et al. (Curr Eye Res, (2004) 28:25-36) have recently shown that contact lenses made with a silicone hydrogel material exhibit much less protein deposition than conventional material. As a minimum, one would hope that an antimicrobial coating would not increase protein and lipid deposition on the underlying material. For Se, this seems to be the case. However, rabbit tears are markedly different from human tears; rabbit tears are much higher in lipid content than human tears. This difference in the chemical composition of rabbit versus human tears leaves open the possibility that the deposition tests run on Se-coated lenses removed from human eyes might show a beneficial (or possibly detrimental) effect. Finally, a few studies suggest that some deposition of tear-film components onto the contact lens surface might actually have a beneficial effect. For example, Cheng et al. (Curr Eye Res. (2004) 28:93-108) have shown that the adsorption of some protein onto the surface of the silicone hydrogel material improves the wettability of the contact lens.

Safety: When evaluating a drug or device for use on humans, determining its safety profile is just as important as investigating whether its intended effect was achieved. A combination of clinical observations and laboratory techniques were used to evaluate the safety of the proposed Se coating on the rabbit eye.

The first clinical technique was standard slit-lamp observation with and without fluorescein after the contact lenses had been removed. Fluorescein staining, hyperemia, neovascularization, edema, infiltrates, and iritis were all evaluated. Of these signs, only punctate and/or arcuate fluorescein staining was found in either the treated or control eye. When punctate staining appeared, the discrete punctate stains always numbered less than 4 per eye and were thus rated trace. There was no difference in the frequency of punctate staining between the eyes that wore the Se-coated lens and eyes that wore the control lens. Occasional arcuate stains appeared that were somewhat random in location and orientation. These covered a much large area than a single punctate stain but the fluorescein pickup was not dense. The radius of the arcuate stains was similar to the radius of a human finger tip or finger nail and, thus, this staining could be simply due to minor contact lens removal trauma on the occasionally spirited rabbit. Whereas the frequency of the occasional arcuate stains was low, their severity was rated as mild. Thus, including these arcuate stains in the data increased both the mean and variance of the staining data.

Pachymetry was used as an objective clinical test that measured either tissue loss or swelling due to edema. If the amount of Se that coated the contact lenses is toxic to corneal cells, it might increase the incidence of cell death or sloughing and cause thinning. On the other hand, if the metabolic pumps that deterge the cornea are somehow adversely affected, the corneas could swell due to edema. There was no evidence for either scenario in our epithelial thickness or total corneal thickness data. The epithelial and total thickness data were not significantly different between experimental and control eyes after 14 days of continuous contact lens wear. However, both the epithelial thickness and total thickness data showed the previously reported slight thinning caused by extended contact lens wear (Holden et al. (1985) Invest Ophthalmol Vis Sci, 26:1489-1501; and Ren et al. (1999) J CLAO, 25:80-100). This later information suggests that the instrument and technique described herein produced adequate resolution to detect a difference between the experimental and control corneas had it been present.

Scanning electron microscopy also revealed no difference between corneas that wore a Se-coated contact lens and a cornea that wore an uncoated contact lens. The gap junctions between cells were tight in all micrographs taken from both experimental and control corneas. In addition, the microvilli appeared unaffected in all micrographs from both groups. There was no evidence of increased shedding in the experimental corneas as assessed by the frequency of younger cells versus older cells. Smaller and lighter cells are thought to be newly emerged younger cells, whereas older cells are darker and larger. Thus, the surface of the rabbit corneal epithelium seemed to be unaffected by the Se coating, even under very high magnification.

Finally, corneas were evaluated histologically at the end of 2 months of continuous wear. None of the experimental or control corneas showed an effect from the contact lens wear. In particular, the corneal epithelium and endothelium appeared completely normal. This also presents strong evidence that the Se coating created no damage to the rabbit corneas.

The data from this pilot study indicates that a Se coating on contact lenses should make contact lens wear freer from acute red eye and bacterial ulceration due to an inhibition of bacterial colonization. In addition, the safety tests suggest that this positive effect could be produced without an adverse effect on corneal health. Finally, the positive results of this study suggest that there may be many other uses of this technology. For example, recent reports of bacterial keratitis and ulcers in orthokeratology or corneal refractive therapy patients suggest that Se coatings could be of value with rigid gas permeable extended wear lenses as well.

### Example 2: Cellulose Sponges - Cell Growth Inhibition

The ability of selenium-treated sponges to modulate the growth of human fibroblasts was conducted utilizing a covalently linked, protected selenium compound to a cellulose Week sponge. Lucigenin-dependent chemiluminesence was used to semi-quantitate the selenium content and the production of superoxide generated by the covalently attached selenocyanatopropionic acid (-CN is the protecting group).

Covalent selenium and control sponges were both tested for their ability to inhibit cellular growth in vitro. The activity of the selenium-coated sponges was tested by measuring incorporation of a radiolabel, tritiated-thymidine, into DNA of cells growing adjacent to the sponge. Human fibroblast cells were grown to approximately 75% confluency in culture and then serum deprived (0% Fetal Calf Serum) for 4 days. At the end of the four days, sponges with and without covalently attached selenium were suspended above human fibroblasts in tissue culture by means of a filter insert placed in the tissue culture well. These fibroblast cells are highly proliferative and a good model for aggressive cell proliferation and invasiveness. The cells were then treated with 10% Fetal Calf Serum for 24 hours, and ³H-thymidine was added at the end of the 24 hours; 24 hours later, the cells were stopped and counted for incorporation of thymidine in DNA.

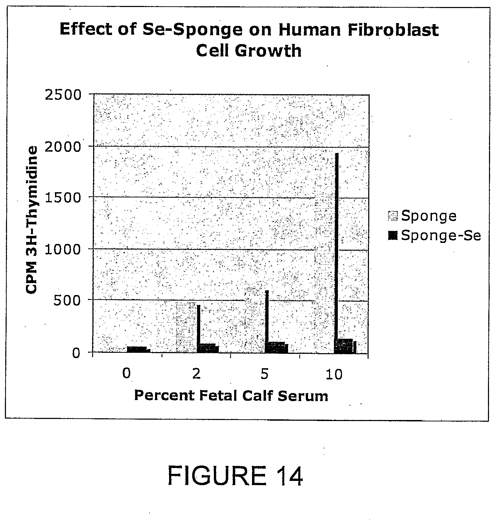

FIG. 14 shows the results obtained with fibroblasts by varying concentrations of calf serum in the presence of sponges containing covalently linked, protected selenium, where cell growth is monitored by incorporation of the tritiated-thymidine into cellular DNA. As can be seen, the sponges are very effective in blocking the growth of fibroblasts in the presence of 10% serum, which contained several different growth factors.

### Example 3: Selenium Attachment to Intraocular Lenses (IQLs)

The ability to non-covalently attach a selenium compound of the present invention to a plastic was demonstrated using an intraocular lens (IOLs). An intraocular lens (or IOL) is an implanted lens in the eye, usually replacing the existing crystalline lens because it has been clouded over by a cataract, or as an alternative to refractive surgery when this procedure is contraindicated. IOLs are typically formed of a plastic such as but not limited to, acrylic, silicone or polymethyl methacrylate (PMMA).

In this study, 6 mg/ml of diphenyl diselenide (DPDS) was added to the IOL, and the mixture was heated at 50-52.5°C for 30 minutes. The treated IOLs were then removed from the solution and allowed to dry. Table IV demonstrates that this procedure resulted in a lens that produced chemiluminescence with no significant leaching of the selenium compound, and therefore temperature and mixing achieved selenium labeling by hydrophobic inclusion.

This demonstrated that the solution without DTT (the unreduced solution) caused more uptake of the non-covalent DPDS, and that none of the non-covalently attached DPDS was leaching out.

**TABLE IV**

| Final Concentration DPDS | DTT Present?* | Chemiluminescence (counts/10 sec)** |
|---|---|---|
| 6 mg/ml | Yes | 28 |
| 3 mg/ml | Yes | 33 |
| 1.5 mg/ml | Yes | 26 |
| 6 mg/ml | No | 48 |
| 3 mg/ml | No | 37 |
| 1.5g/ml | No | 92 |

| | | |
|---|---|---|
| *Presence of DTT reduced **Background level of chemiluminescence was 20 counts/10 sec. | | |

In addition, oxygen treatment of the IOLs using a plasma treatment allowed selenium attachment to an IOL plastic using a protected selenium compound. The silicone IOL was placed in a plasma chamber and then treated with plasma in the presence of Oxygen gas. The lens was then placed in a buffer solution at pH 5.8 with EDC (1-ethyl-3-[3-dimethylaminopropyl]carbodiimide) and sulfo-NHS (N-hydroxysulfosuccinimide), and the carboxyl residues created on the surface of the lens couple with the succinimide group. This activated lens was then reacted with Selenocyanatopropyl amine.

The experimental design for this procedure was as follows:

The IOL was activated by washing in analytical-grade water, placing in a well of a tissue culture plate, and adding 1 ml of a solution prepared by dissolving 540 mg sulfo-NHS and 480 mg EDC in 100ml 0.25M, pH 6.0 MES buffer. The plate was placed on a shaker and allowed to shake constantly for 2 hours, and then each IOL was washed with analytical-grade water 3 times.

A selenocystamine solution was prepared by dissolving 400 mg selenocystamine.2HCL in 40ml water and 40ml ethanol, and a solution of 210mg NaHCO₃ in 20ml water was then added into the above solution.

Coupling of Selenium compound to the IOL was performed by transferring 1 ml of the above selenocystamine solution into the tissue culture plate wells. The plates were kept in the dark by use of aluminum foil, and allowed to constantly shake overnight (12 hours minimum). The solution was then removed and the lenses treated with 2 ml of 0.15 mM glutathione (4.6 mg/100ml normal saline) for one hour. The IOLs were then washed two times with ethanol and two times with water. The IOLs were then put into bottles containing 2 ml of normal saline and autoclaved.

The chemiluminescence results for this experiment are shown in Table V.

**Table V. Chemiluminescence Results of Plasma Treatment of IOL with Protected Selenium Compound**

| | Counts | Background |
|---|---|---|
| Oxygen-cathode | 468 | 35 |
| Oxygen - anode | 122 | 15 |

### Example 4: Hydrophobic Inclusion of the Selenium Compounds into Silicon and Acrylics

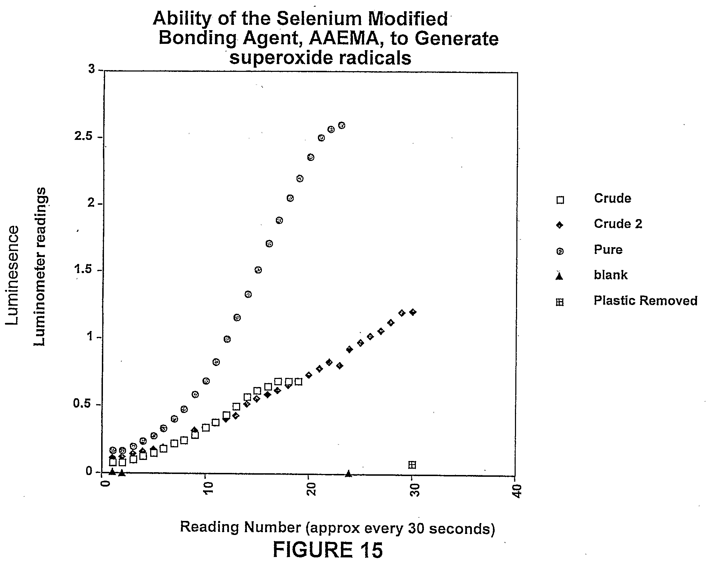

In addition to the hydrophobic inclusion experiments described above, hydrophobic inclusion of a protected selenium compound into a bonding agent was also demonstrated. In this hydrophobic mixing experiment, a selenium compound comprising a CN protecting group [2-(selenocyanatoacetoxy)butoxyethyl methacrylate] was mixed with the bonding agent 2- (Acetoacetoxy)ethylmethacrylate (AAEMA) to a final selenium concentration of 5%. The selenium composition was then applied to a surface, such as a PMMA plastic or a human toenail (results with both surfaces were the same), preferably at a concentration of about 100 mg/cm². The ability of the selenium modified bonding agent to generate superoxide radicals was then measured by chemiluminescence, as shown in FIG. 15. In FIG. 15, "Crude 1" and "Crude 2" refer to a mixture of the selenium labeled material with AAEMA; "pure" refers to the pure selenium labeled compound (no AAEMA); "blank" refers to the AAEMA with no selenium; for "plastic removed", the mixture-coated plastic was soaked in water, and then after removal of the plastic, the aqueous solution was tested for any selenium that may have come off. This experiment demonstrates that the protected selenium compound can be mixed with a bonding agent and coated onto a surface and allowed to dry and still demonstrate chemiluminescence with no significant leaching. The fact that there was very little if any counts present in the "plastic removed" sample indicates that the selenium compound remained associated with the AAEMA treated plastic.

### Technique 6: Inhibition of HIV with Protected Selenium-Peptide Compound

For this experiment, HIV was incubated with a protected selenopeptide comprising selenocyanatopropionic acid attached to the amino group on the amino terminal threonine of the following peptide: T-Y-I-Cbzl-Ebzl-V-E-D-Q-K-E-E; where T = threonine, Y = Tyrosine, I = Isoleucine, C = cysteine, E = glutamic acid, v = valine, D = aspartic acid, Q = glutamine, K = lysine; bzl is a benzyl protecting group (on cysteine it is an S-benzyl group). HIV was incubated with the protected selenopeptide for 2 hours at 0.1 [mu]g/ml, 1µg/ml, 10 µg/ml and 100 µg/ml. Glutathione was added to the solution to activate the virus, and at the end of that time the virus was diluted and then allowed to infect appropriate cells growing in culture. The number of cells infected was determined by syncytia formation. This provided a measure of the number of infective viral particles.

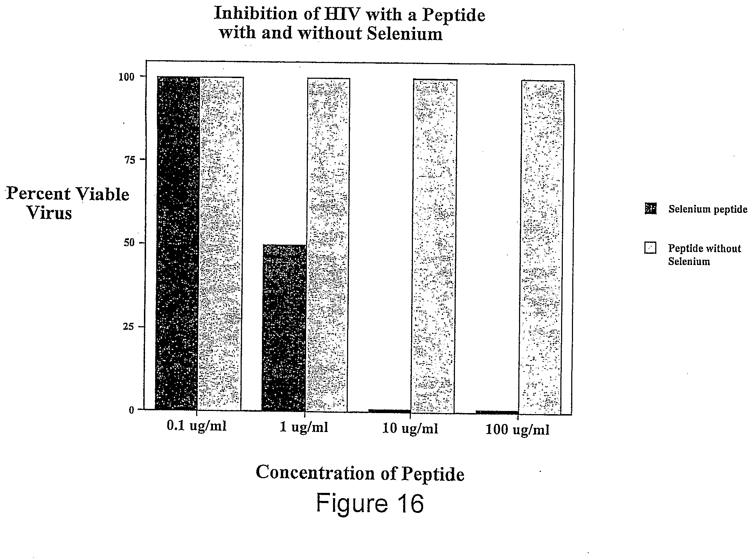

The resulting data are shown in FIG. 16. This data shows that a low concentration of selenopeptide (10 µM) can inactivate HIV in a short period of time.

### Example 5: Selenium Compounds with Different Protecting Groups

It is important that the protecting groups attached to the selenium compounds of the present invention be easily removed by compounds such as thiols, because the protecting groups allow for the synthesis of seleno-drugs, seleno-devices or seleno-coatings, that are not di-selenides. Di-selenides can cause crosslinking and release a toxic selenium compound from the drug, device or coating. In contrast, the removal of the protecting groups disclosed and claimed herein does not result in production of a toxic compound, thus demonstrating one of the advantages of the selenium compounds of the present invention.

Extensive tests have been carried out with cyanate as a protecting group and have been described herein above in the previous Examples. Additioonal tests were also carried out with other potential protecting groups such as halides (ie. chloride, bromide, iodide), nitrogen containing compounds such as N-(phenylseleno)phthalimide (NPPD), and the mixed anhydrides such as the one between phenyl seleninic acid and acetic acid (PSAA). Table VI contains data that demonstrates that selenium compounds containing these protecting groups are capable of being activated by the presence of thiols similar to those found in in vivo settings and thus generate superoxide radicals.

Table VI shows the ability of these additional classes of protected selenium compounds to generate superoxide radicals in aqueous solution even though they are not very soluble in water above a background of 1-2 cpm.

**TABLE VI. Activity of Selenium Compounds with Different Protecting Groups**

| Compound | Counts/min Superoxide Formation |
|---|---|
| phenyl selenochloride | 724 |
| phenyl selenobromide | 830 |
| phenyl selenoiodide | 1130 |
| NPPD | 84 |
| PSAA | 93 |
| selenocyanatopropionic acid | 2924 |

Another class of protecting groups that fall within the scope of the present invention are water soluble protecting groups. Examples of water soluble protecting groups include, but are not limited to, sulfate, nitrate, phosphate and carbonate attached to the selenium of the organo-selenium molecule of the present invention. These water soluble protecting groups are important because of their ability to confer water solubility to the organo-selenium compounds of the present invention. Table VII illustrates that an organo-selenium compound containing sulfate as the protecting group can be activated with glutathione. For this experiment, sulfo-selenoethyl amine (NH₂-CH₂-CH₂-Se-SO₃H₂) was dissolved in buffer containing glutathione (100 uM) and Lucigenin at pH 7.4, and this data demonstrates that this compound can be activated with glutathione. Other water soluble protecting groups would function in a similar manner, and thus are included within the scope of the present invention.

**TABLE VII.**

| Compound | chemiluminescence (cpm) |
|---|---|
| Sulfo-selenoethyl amine | 562 |
| Blank | 36 |

In addition, a selenium compound containing a CN protecting group was covalently attached to silicon to form trimethylsilylselenocyanate. The chemiluminescence (CL) of this compound was measured at 30 second intervals, pH 7.3 and 36°C. The activity of this compound is shown in Table VIII.

**TABLE VIII.**

| Time elapsed (minutes) | Chemiluminescence |
|---|---|
| 0.5 | 99 |
| 1.5 | 474 |
| 2.0 | 431 |
| 2.5 | 408 |
| 3.0 | 382 |
| 3.5 | 343 |
| 4.0 | 290 |
| 4.5 | 268 |
| 5.0 | 262 |
| 5.5 | 228 |

The activity of this compound demonstrates the ability to attach a protected form of selenium to silicone and retain activity to generate superoxide radicals. Thus, these results demonstrate a new class of compounds that could be used for biomaterials and drugs.

Thus, in accordance with the present invention, there has been provided a method of producing selenium-based biocidal formulations that fully satisfies the objectives and advantages set forth hereinabove.

## Claims

1. A method for preventing growth of a living cell and/or a living organism on a solid substrate, comprising the steps of:
providing a solid substrate, wherein the solid substrate is not a human or animal body;
applying a polymer matrix comprising an effective amount of a selenium compound to the solid substrate, wherein the selenium compound is selected from the group consisting of RSeH, RSeR', RSeSeR, RSeSeR', and RSeX, wherein R and R' are each an aliphatic or phenolic residue, wherein R comprises a reactive group that binds the selenium compound to the solid substrate via the polymer matrix, and wherein X is a protecting group selected from the group consisting of -O-R, -S-R, a halogen, an imide, a cyanide, an azide, a phosphate, a sulfate, a nitrate, a carbonate, and selenium dioxide, and wherein the selenium compound is non-covalently associated with the solid substrate via the polymer matrix; and
wherein when the selenium compound comes into contact with and binds to a surface of the living cell and/or living organism, the selenium anion Se- and free radical species are formed, the free radical species inhibits and/or inactivates said living cell and/or living organism, thereby preventing growth of the living cell and/or living organism on the solid substrate, and wherein the selenium anion Se- remains attached to the surface of the solid substrate via the polymer matrix after generation of the selenium anion Se- and the free radical species, such that selenium anion can continually generate additional free radical species when brought into contact with additional living cells and/or organisms.

2. The method of claim 1 wherein the living cell and/or a living organism is selected from the group consisting of bacteria, viruses, mold, fungi, protozoa parasites, plant cells, animal cells, biological materials and combinations thereof.

3. The method of claim 1, wherein the selenium composition comprises a formula selected from the group consisting of R-Se-CN, R-Se-Cl, R-Se-Br, R-Se-I, R-Se-N₃, R-Se-S-R, and R-Se-O-R.

4. The method of claim 1 wherein the effective amount of the selenium compound is in a range of from 0.01 µg to 100 µg of elemental selenium per square centimeter of surface area of the solid substrate.

5. The method of claim 1, wherein the step of applying an effective amount of a selenium compound to a solid substrate is further defined as incorporating the selenium compound into a coating composition that is applied to the solid substrate.

6. The method of claim 1, wherein the polymer matrix comprises 2-(acetoacetoxy)ethyl methacrylate.

7. The method of claim 1, wherein each of R and R' is an aliphatic residue containing at least one reactive group selected from the group consisting of aldehyde, carboxylic, amino, alcoholic, phosphate, sulfate, halogen, phenolic reactive groups and combinations thereof.

8. The method of claim 1, wherein R is selected from the group consisting of-(CH₂)ₙHN₂, --(CH₂)ₙCOOH, and --(CH₂)ₙ--Ø, wherein n is an integer greater than 1.

9. The method of claim 1, wherein the selenium compound is selected from the group consisting of NH₂CH₂CH₂SeCH₃ (RSeR'), NH₂CH₂CH₂SeCH₂CH₂NH₂ (RSeR), NH₂CH₂CH₂SeSeCH₂CH₂NH₂ (RSeSeR), NH₂CH₂CH₂SeSeCH₂CH₂NH-cellulose (RseSeR'), and NH₂CH₂CH₂SeCN (RSeX).

10. The method of claim 1, wherein the growth prevention occurs at 4°C.

11. The method of claim 1, wherein the living cell and/or organism comprises an infectious agent.

12. The method of claim 1 wherein the polymer matrix comprises an effective amount of the selenium compound selenocyanatoacetoxy butoxyethyl methacrylate.

## Patentansprüche

1. Verfahren zum Verhindern des Wachstums einer lebenden Zelle und/oder eines lebenden Organismus auf einem festen Substrat, wobei das Verfahren die folgenden Schritte umfasst:
Bereitstellen eines festen Substrats, wobei das feste Substrat kein menschlicher oder tierischer Körper ist;
Auftragen einer polymeren Matrix, die eine wirksame Menge einer Selenverbindung umfasst, auf das feste Substrat, wobei die Selenverbindung ausgewählt ist aus der Gruppe bestehend aus RSeH, RSeR', RSeSeR, RSeSeR' und RSeX, wobei R und R' jeweils ein aliphatischer oder phenolischer Rest sind, wobei R eine reaktionsfähige Gruppe umfasst, welche die Selenverbindung über die polymere Matrix an das feste Substrat bindet, und wobei X eine Schutzgruppe ist, die ausgewählt ist aus der Gruppe bestehend aus -O-R, -S-R, einem Halogen, einem Imid, einem Cyanid, einem Azid, einem Phosphat, einem Sulfat, einem Nitrat, einem Carbonat und Selendioxid, und wobei die Selenverbindung mit dem festen Substrat über die polymere Matrix nicht kovalent assoziiert ist; und
wobei, wenn die Selenverbindung in Kontakt mit einer Oberfläche der lebenden Zelle und/oder des lebenden Organismus gelangt und damit eine Verbindung eingeht, das Selenanion Se- und freie radikale Spezies gebildet werden, wobei die freien radikalen Spezies die lebende Zelle und/oder den lebenden Organismus hemmen und/oder inaktivieren, wodurch das Wachstum der lebenden Zelle und/oder des lebenden Organismus auf dem festen Substrat verhindert wird, und wobei das Selenanion Se- über die polymere Matrix nach der Erzeugung des Selenanions Se- und der freien radikalen Spezies weiter an der Oberfläche des festen Substrats anhaftet, so dass das Selenanion fortgesetzt weitere freie radikale Spezies erzeugen kann, wenn es mit weiteren lebenden Zellen und/oder Organismen in Kontakt gebracht wird.

2. Verfahren nach Anspruch 1, wobei die lebende Zelle und/oder ein lebender Organismus ausgewählt ist aus der Gruppe bestehend aus Bakterien, Viren, Schimmel, Pilzen, Protozoen-Parasiten, Pflanzenzellen, tierischen Zellen, biologischen Substanzen und Kombinationen dieser.

3. Verfahren nach Anspruch 1, wobei die Selenzusammensetzung eine Formel umfasst, die ausgewählt ist aus der Gruppe bestehend aus R-Se-CN, R-Se-Cl, R-Se-Br, R-Se-I, R-Se-N₃, R-Se-S-R und R-Se-O-R.

4. Verfahren nach Anspruch 1, wobei die wirksame Menge der Selenverbindung in einem Bereich von 0,01 µg bis 100 µg elementares Selen je Quadratzentimeter der Oberfläche des festen Substrats liegt.

5. Verfahren nach Anspruch 1, wobei der Schritt des Auftragens einer wirksamen Menge einer Selenverbindung auf ein festes Substrat ferner definiert ist als Inkorporieren der Selenverbindung in eine Überzugszusammensetzung, die auf das feste Substrat aufgetragen wird.

6. Verfahren nach Anspruch 1, wobei die polymere Matrix 2-(Acetoacetoxy)ethylmethacrylat umfasst.

7. Verfahren nach Anspruch 1, wobei jedes R und R' ein aliphatischer Rest ist, der mindestens eine reaktionsfähige Gruppe enthält, die ausgewählt ist aus der Gruppe bestehend aus reaktionsfähigen Aldehyd-, Carboxyl-, Amino-, Alkohol-, Phosphat-, Sulfat-, Halogen-, Phenol-Gruppen und Kombinationen dieser.

8. Verfahren nach Anspruch 1, wobei R ausgewählt ist aus der Gruppe bestehend aus --(CH₂)HN₂, --(CH₂)ₙCOOH und --(CH₂)ₙ--Ø, wobei n eine ganze Zahl größer 1 ist.

9. Verfahren nach Anspruch 1, wobei die Selenverbindung ausgewählt ist aus der Gruppe bestehend aus NH₂-CH₂-SeCH₃(RSeR'), NH₂CH₂CH₂SeCH₂CH₂NH₂ (RSeR), NH₂CH₂CH₂SeSeCH₂CH₂NH₂(RSeSeR), NH₂CH₂CH₂SeSeCH₂CH₂NH-Cellulose (RseSeR') und NH₂CH₂CH₂SeCN (RSeX).

10. Verfahren nach Anspruch 1, wobei die Wachstumsprävention bei 4 °C eintritt.

11. Verfahren nach Anspruch 1, wobei die lebende Zelle und/oder der lebende Organismus einen Infektionserreger umfasst.

12. Verfahren nach Anspruch 1, wobei die polymere Matrix eine wirksame Menge der Selenverbindung Selenocyanatoacetoxybutoxyethylmethacrylat umfasst.

## Revendications

1. Procédé pour empêcher la croissance d'une cellule vivante et/ou d'un organisme vivant sur un substrat solide, comprenant les étapes consistant à :
fournir un substrat solide, le substrat solide n'étant pas un corps humain ou animal ;
appliquer une matrice polymère comprenant une quantité efficace d'un composé de sélénium sur le substrat solide, le composé de sélénium étant choisi dans le groupe constitué par RSeH, RSeR', RSeSeR, RSeSeR'et RSeX, R et R' étant chacun un résidu aliphatique ou phénolique, R comprenant un groupe réactif qui lie le composé de sélénium au substrat solide par l'intermédiaire de la matrice polymère, et X étant un groupe protecteur choisi dans le groupe constitué par -O-R, -S-R, un halogène, un imide, un cyanure, un azide, un phosphate, un sulfate, un nitrate, un carbonate et du dioxyde de sélénium, et le composé du sélénium étant associé de manière non-covalente au substrat solide par l'intermédiaire de la matrice polymère ; et
lorsque le composé de sélénium entre en contact avec une surface de la cellule vivante et/ou de l'organisme vivant et se lie à une telle surface, l'anion de sélénium Se- et des espèces de radicaux libres étant formées, les espèces de radicaux libres inhibant et/ou inactivant ladite cellule vivante et/ou ledit organisme vivant, empêchant ainsi la croissance de la cellule vivante et/ou de l'organisme vivant sur le substrat solide, et l'anion de sélénium Se- restant fixé à la surface du substrat solide par l'intermédiaire de la matrice polymère après la génération de l'anion de sélénium Se- et des espèces de radicaux libres, de sorte que l'anion de sélénium puisse générer en continu d'autres espèces de radicaux libres lorsqu'il est mis en contact avec d'autres cellules vivantes et/ou organismes vivants.

2. Procédé selon la revendication 1, la cellule vivante et/ou un organisme vivant étant choisi dans le groupe constitué par les bactéries, les virus, les moisissures, les champignons, les parasites protozoaires, les cellules végétales, les cellules animales, les matières biologiques et leurs combinaisons.

3. Procédé selon la revendication 1, la composition de sélénium comprenant une formule choisie dans le groupe constitué par R-Se-CN, R-Se-Cl, R-Se-Br, R-Se-I, R-Se-N₃, R-Se-S-R et R-Se-O-R.

4. Procédé selon la revendication 1, la quantité efficace du composé de sélénium se situant dans une plage comprise entre 0,01 µg et 100 µg de sélénium élémentaire par centimètre carré de surface du substrat solide.

5. Procédé selon la revendication 1, l'application d'une quantité efficace d'un composé de sélénium sur un substrat solide étant en outre définie comme incorporant le composé de sélénium dans une composition de revêtement qui est appliquée sur le substrat solide.

6. Procédé selon la revendication 1, la matrice polymère comprenant du 2-(acétoacétoxy)éthyle méthacrylate.

7. Procédé selon la revendication 1, R et R' représentant chacun un résidu aliphatique contenant au moins un groupe réactif choisi dans le groupe constitué par les groupes réactifs aldéhyde, carboxylique, amino, alcoolique, phosphate, sulfate, halogène, phénolique et leurs combinaisons.

8. Procédé selon la revendication 1, R étant choisi dans le groupe constitué par-(CH₂)ₙHN₂, --(CH₂)ₙCOOH, et --(CH₂)ₙ--Ø, n étant un entier supérieur à 1.

9. Procédé selon la revendication 1, le composé de sélénium étant choisi dans le groupe constitué par NH₂CH₂CH₂SeCH₃(RSeR'), NH₂CH₂CH₂SeCH₂CH₂NH₂(RSeR), NH₂CH₂CH₂SeSeCH₂CH₂NH₂ (RSeSeR), NH₂CH₂CH₂SeSeCH₂CH₂NH-cellulose (RseSeR'), et NH₂CH₂CH₂SeCN (RSeX).

10. Procédé selon la revendication 1, la prévention de la croissance se produisant à 4 °C.

11. Procédé selon la revendication 1, la cellule vivante et/ou l'organisme vivant comprenant un agent infectieux.

12. Procédé selon la revendication 1, la matrice polymère comprenant une quantité efficace du composé de sélénium sélénocyanatoacétoxy butoxyéthyl méthacrylate.
